# EUROPEAN PATENT APPLICATION

(11) **EP 3 168 224 A1**
(43) Date of publication of application: **17.05.2017**
(21) Application number: 15194823.9
(22) Date of filing: 16.11.2015
(51) Int. Cl.: C07F 19/00, C07F 3/06, C07B 37/04, C07C 201/12, C07D 239/30

(54) **ORGANOZINC COMPOSITION**

(71) Applicant: Ludwig-Maximilians-Universität München, 80539 München (DE)
(72) Inventor: Knochel, Paul, 81479 München (DE); Becker, Matthias, 82131 Gauting (DE)
(74) Representative: Vossius & Partner Patentanwälte Rechtsanwälte mbB

(57) **Abstract**

The present invention relates to compositions containing a zinc complex wherein a zinc atom carries two amide ligands, a lithium salt and a defined solvent. The compositions are stable and show high activity as reactants for metalation reactions, and thus provide a valuable tool in organic synthesis. Moreover, the invention relates to processes for the preparation of the compositions and to synthetic procedures using them.

## Description

The present invention relates to compositions containing a zinc amide base which are stable and show high activity as reactants for metalation reactions, and thus provide a valuable tool in organic synthesis. Moreover, the invention relates to processes for the preparation of the compositions and to synthetic procedures using them.

Monoamide bases of the form R₂N-MetX, wherein R is an organic group, Met is Zn and X is a suitable anion, have been developed as reagents for the metalation of organic compounds (cf. WO 2010/092096 A1 and the literature cited therein). However, especially amide ligands derived from amines which can be conveniently obtained at moderate costs, e.g. for R being isopropyl, trimethylsilyl, or cyclohexyl show, also under conditions of the continuous flow reaction, only a limited solubility in conventional reaction media such as thetrahydrofurane or diethyl ether, and insufficient reactivity in the metalation of substrates such as unsaturated substrates containing a Lewis-acidic hydrogen atom. Similar limitations were encountered for bisamide bases of the structure R₂N-Met-NR₂ (cf. S. Wunderlich, Dissertation, Ludwig-Maximilians-Universitat, München 2010).

Thus, the objective underlying the present invention was to provide a stable composition containing a soluble organozinc complex, which is versatile with respect to the chemical nature of the ligands bound in the organozinc complex, ensures a high activity of the organozinc complex in metalation reactions and allows also amides to be used which are obtainable at moderate costs.

To that extent, the present invention provides, in a first aspect, an organozinc composition comprising:
(a) an zinc complex, wherein a zinc atom carries two identical or different amide ligands of formula (I), which amide ligands are bound to the zinc atom via their nitrogen atom:

   R^{a}R^{b}N (I)

   wherein R^{a} and R^{b} are independently selected from a saturated C₁₋₁₀ hydrocarbon group, and wherein optionally one or more of the carbon atoms in the hydrocarbon group may be replaced by a silicon atom each; a group R^{a} and a group R^{b} may be connected in the same ligand R^{a}R^{b}N to form a 5 to 8 membered ring together with the nitrogen atom to which they are attached; and/or
   a group R^{a} or R^{b} of one amide ligand may be connected with a group R^{a} or R^{b} of the other amide ligand, such that a bidentate ligand comprising two amide groups is formed;
(b) a lithium salt of the formula LiX,
   wherein
   X is an anion selected from the group consisting of H⁻, F⁻, Cl⁻, Br⁻, I⁻, SCN⁻, CN⁻, NCO⁻, HalOₘ⁻, wherein Hal is selected from Cl, Br, and I and m is 3 or 4, NOₚ⁻, wherein p is 2 or 3, BF₄⁻, PF₆⁻, R'O⁻, R'C(O)O⁻, R'S⁻, R'C(O)S⁻, R'C(S)O⁻, R'C(S)S⁻, R'R"N⁻, R'(R"O)P(O)O⁻, R'O(R"O)P(O)O⁻, R'R"P⁻, R'SO₃⁻, R'SO₂S⁻, and R'OSO₃⁻, wherein R' is H or an organic group selected from aryl, heteroaryl, alkaryl, alkyl, cycloalkyl, alkenyl, and alkynyl, which organic group may be optionally substituted, wherein R" is H or an organic group selected from aryl, heteroaryl, alkaryl, alkyl, cycloalkyl, alkenyl and alkynyl, which organic group may be optionally substituted, and wherein two suitable organic groups R' and R" may be connected to form a ring together with the atoms to which they are attached;
(c) a solvent S1 or a solvent mixture comprising at least one solvent S1, wherein S1 is a solvent of formula (II) : 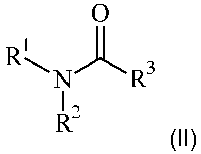 wherein
   R¹ is selected from H and a saturated C₁₋₁₀ hydrocarbon group;
   R² is a saturated C₁₋₁₀ hydrocarbon group;
   and R³ is selected from NR⁴R⁵, H and R⁶, wherein R⁴ is selected from H and a saturated C₁₋₁₀ hydrocarbon group, R⁵ is a saturated C₁₋₁₀ hydrocarbon group and R⁶ is a saturated C₁₋₁₀ hydrocarbon group;
   and wherein
   R² and R⁵ or R² and R⁶ may be connected to form a 5 to 8 membered ring together with the atoms to which they are attached.

It has been found that the combination of a zinc-bisamide complex (a), the lithium salt (b) and the solvent or solvent mixture (c) provides a high stability and a high solubility of the zinc amide complex and ensures a high activity of the composition in accordance with the invention, in particular in metalation reactions. Surprisingly, these effects are particularly pronounced for zinc amide complexes carrying amide ligands which are derived from simple amines, i.e. amines obtainable at moderate costs for which zinc complexes with a high activity had not previously been available. Moreover, the composition in accordance with the invention allows the metalation of substrates carrying sensitive functional groups.

In a further aspect of the invention, a process for the preparation of the above organozinc composition is provided.

Still further aspects of the invention are concerned with the application of the organozinc composition in accordance with the invention. In particular, the organozinc composition can be suitably used in a process for the preparation of a broad variety of organozinc compounds. Moreover, processes are encompassed wherein an organozinc composition prepared in accordance with the invention is reacted with an electrophilic reactant or with a reactant containing an electrophilic moiety, or wherein an organometallic compound containing a metal other than Zn is formed in a transmetallation reaction by the reaction with an organozinc composition prepared in accordance with the invention.

### Component (a): organozinc complex

Component (a) of the composition in accordance with the present invention is an organozinc complex wherein a zinc atom carries two identical or different amide ligands of formula (I), which amide ligands are bound to the zinc atom via their nitrogen atom:

R^{a}R^{b}N (I)

R^{a} and R^{b} are independently selected from a saturated C₁₋₁₀ hydrocarbon group. Optionally, one or more of the carbon atoms in the hydrocarbon group may be replaced by a silicon atom each, and/or a group R^{a} and a group R^{b} may be connected in the same ligand R^{a}R^{b}N to form a 5 to 8 membered ring together with the nitrogen atom to which they are attached, and/or a group R^{a} or R^{b} of one amide ligand may be connected with a group R^{a} or R^{b} of the other amide ligand, such that a bidentate ligand comprising two amide groups is formed. The saturated hydrocarbon group may be linear, branched or cyclic, or may combine linear, branched or cyclic moieties.

The composition of the organozinc complex can be represented as Zn(NR^{a}R^{b})₂. However, as will be understood, this formula defines the relationship between the zinc atoms and the amide ligands, without excluding the possibility of further coordinative bonds (e.g. by other components of the composition) being formed.

Preferably, R^{a} and R^{b} are independently selected from a linear or branched C₁₋₁₀ alkyl group and a C₃₋₈ cycloalkyl group (including combinations thereof wherein an alkyl group carries a cycloalkyl substituent, or cycloalkyl group carries one or more alkyl substituents), a C₃₋₁₀ trialkylsilyl group (wherein C₃₋₁₀ designates the total number of carbon atoms in the alkyl moieties, also hereinafter), and a structure wherein a group R^{a} and a group R^{b} are connected in the same ligand R^{a}R^{b}N form a 2,2,6,6-tetramethylpiperidyl ring together with the nitrogen atom to which they are attached.

More preferably, at least one of R^{a} and R^{b} in each of the two amide ligands, preferably both R^{a} and R^{b}, are independently selected from a branched C₃₋₇, alkyl group, a C₅₋₇, cycloalkyl group, and a C₃₋₁₀ trialkylsilyl group. Even more preferably, R^{a} and R^{b} are independently selected from cyclohexyl, isopropyl, trimethylsilyl, and *t*-butyl, and most preferably R^{a} and R^{b} are both cyclohexyl.

As will be understood, the coordinative bond between the zinc atom and the ligand amide is typically a σ-bond. It is preferred that the amide ligands are bound as terminal ligands to the zinc atom.

In the zinc complex of component (a), a zinc atom carries two amide ligands. As will be understood by the skilled reader, this encompasses the possibility that further coordinative bonds are formed to the zinc atom to lead to a higher coordination number, e.g. by a coordinating anion X provided by the lithium salt of component (b) discussed below, or by a solvent molecule.

### Component (b): lithium salt

Component (b) in accordance with the present invention is a lithium salt of the formula LiX. In this salt, X is an anion selected from the group consisting of H⁻, F⁻, Cl⁻, Br⁻, I⁻, SCN⁻, CN⁻, NCO⁻, HalOₘ⁻, wherein Hal is selected from Cl, Br, and I and m is 3 or 4, NOₚ⁻, wherein p is 2 or 3, BF₄⁻, PF₆⁻, R'O⁻, R'C(O)O⁻, R'S⁻, R'C(O)S⁻, R'C(S)O⁻, R'C(S)S⁻, R'R"N⁻, R'(R"O)P(O)O⁻, R'O(R"O)P(O)O⁻, R'R"P⁻, R'SO₃⁻, R'SO₂S⁻, and R'OSO₃⁻.

In the above groups, R' is H or an organic group selected from aryl, heteroaryl, alkaryl, alkyl, cycloalkyl, alkenyl and alkynyl, which organic group may be optionally substituted, wherein R" is H or an organic group selected from aryl, heteroaryl, alkaryl, alkyl, cycloalkyl, alkenyl and alkynyl, which organic group may be optionally substituted, and wherein two suitable organic groups R' and R" may be connected to form a ring together with the atoms to which they are attached. Preferably, R' and R" are an organic group as defined above. More preferably, R' and R" are optionally substituted alkyl, or optionally substituted aryl, and most preferably alkyl. The number of carbon atoms in R' and R" is preferably 1 to 7, more preferably 1 to 4.

As exemplary and preferred substituents aryl, heteroaryl, cycloalkyl, or the aryl moiety of an alkaryl group, reference can be made to halogen (i.e. F, Cl, Br, and I), OH, CN, C₁₋₆ alkyl, in particular methyl, C₁₋₆ fluoroalkyl, in particular trifluoromethyl, NO₂, NR^{x}R^{y}, -COOR^{z}, C₁₋₆ alkoxy, or C₁₋₆ alkenyloxy-C₁₋₆ alkoxy, wherein R^{x} and R^{y} are independently selected from H and C₁₋₆ alkyl, and R^{z} is C₁₋₆ alkyl. As exemplary and preferred substituents for an alkyl group, an alkenyl group, an alkynyl group or the alkenyl moiety of an alkaryl group, reference can be made to halogen (i.e. F, Cl, Br, and I), OH, CN, NO₂, NR^{x}R^{y}, -COOR^{z}, C₁₋₆ alkoxy, or C₁₋₆ alkenyloxy-C₁₋₆ alkoxy, wherein R^{x} and R^{y} are independently selected from H and C₁₋₆ alkyl, and R^{z} is C₁₋₆ alkyl. However, unless specifically defined otherwise, it is preferred that the substituents are absent.

Preferably X is an anion selected from the group consisting of F⁻, Cl⁻, Br⁻, I⁻, SCN⁻, CN⁻, NCO⁻, HalOₘ⁻, wherein Hal is selected from Cl, Br, and I and m is 3 or 4, NOₚ⁻, wherein p is 2 or 3, R'O⁻, R'C(O)O⁻, R'S⁻, R'SO₃⁻, R'SO₂S⁻, and R'OSO₃⁻. More preferably, X is an anion selected from the group consisting of Cl⁻, Br⁻, I⁻, SCN⁻, CN⁻, NCO⁻, tosylate (p-tolylsulfonate), mesylate (methanesulfonate) and R'O⁻, wherein R' is C1-4 alkyl. Most preferably, X is Cl⁻.

Without wishing to be bound by theory, it is assumed that the lithium salt facilitates the breaking-up of agglomerates formed by zinc complexes, thus leading to an activation of the complexes.

### Component (c): solvent or solvent mixture

Component (c) in accordance with the present invention is a solvent S1 or a solvent mixture comprising at least one solvent S1, wherein S1 is a solvent of formula (II): 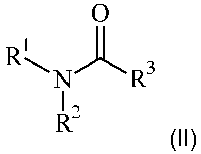 wherein R¹ is selected from H and a saturated C₁₋₁₀ hydrocarbon group; R² is a saturated C₁₋₁₀ hydrocarbon group; and R³ is selected from NR⁴R⁵, H and R⁶, wherein R⁴ is selected from H and a saturated C₁₋₁₀ hydrocarbon group, R⁵ is a saturated C₁₋₁₀ hydrocarbon group and R⁶ is a saturated C₁₋₁₀ hydrocarbon group; and wherein optionally R² and R⁵ or R² and R⁶ may be connected to form a 5 to 8 membered ring together with the atoms to which they are attached. Preferred as a saturated C₁₋₁₀ hydrocarbon group for R², R⁵ and R⁶ is a linear or branched alkyl group. R¹ and R⁴ are preferably selected from H or a linear or branched alkyl group, in particular from H, methyl and ethyl.

Preferably, S1 is a solvent of formula (IIa) or (IIb), more preferably of formula (IIa): 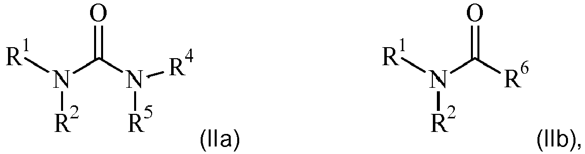 wherein R¹ is selected from H and a saturated C₁₋₁₀ hydrocarbon group, preferably from H and a linear or branched C₁₋₁₀ alkyl group, and more preferably from H, methyl and ethyl; R² is a saturated C₁₋₁₀ hydrocarbon group, preferably a linear or branched C₁₋₁₀ alkyl group; R⁴ is selected from H and a saturated C₁₋₁₀ hydrocarbon group, preferably from H and a linear or branched C₁₋₁₀ alkyl group, and more preferably from H, methyl and ethyl;
R⁵ is a saturated C₁₋₁₀ hydrocarbon group, preferably a linear or branched C₁₋₁₀ alkyl group;
and R⁶ is selected from H and a saturated C₁₋₁₀ hydrocarbon group, preferably from H and a linear or branched C₁₋₁₀ alkyl group;
and wherein optionally R² and R⁵ or R² and R⁶ may be connected to form a 5 to 8 membered ring together with the atoms to which they are attached. Among the preferred solvents S1 of formula (IIa), it is further preferred that the compound of formula (IIa) is a cyclic compound wherein R² and R⁵ are connected to form a 5 to 8 membered ring, more preferably a 5 or 6 membered ring, together with the atoms to which they are attached.

More preferably, R¹ is selected from H, methyl and ethyl, R² is a linear or branched C₁₋₆ alkyl group, R⁴ is selected from H, methyl and ethyl, R⁵ is a linear or branched C₁₋₆ alkyl group, R⁶ is selected from H and a linear or branched C₁₋₆ alkyl group; and optionally R² and R⁵ or R² and R⁶ may be connected to form a 5 or 6 membered ring together with the atoms to which they are attached.

Even more preferably S1 is selected from the group consisting of 1,3-dimethyl-3,4,5,6-tetrahydro-2(1*H*)-pyrimidinone (DMPU), *N*,*N*,*N*',*N*'-tetramethylurea (TMU), *N*-methyl-2-pyrrolidone (NMP), *N*-ethyl-2-pyrrolidone (NEP), and dimethylformamide (DMF). Even more preferably S1 is selected from the group consisting of 1,3-dimethyl-3,4,5,6-tetrahydro-2(1*H*)-pyrimidinone and *N*,*N*,*N*,*N*-tetramethylurea. Most preferably S1 is 1,3-dimethyl-3,4,5,6-tetrahydro-2(1*H*)-pyrimidinone.

It has been surprisingly found that the presence of the solvent S1 allows the preparation of organozinc compositions containing the zinc amide complex in highly active form and/or at increased concentrations.

In the organozinc composition in accordance with the invention, the solvent S1 can be the only solvent, or a solvent mixture comprising at least one solvent S1 and at least one further solvent S2 different from S1 can be present. Preferably, the solvent mixture comprising at least one solvent S1, and at least one further solvent S2 different from S1 is used. It is further preferred that the solvent mixture consists of one solvent S1 and one further solvent S2 different from S1.

Preferably, the solvent S2 is selected from the group consisting of acetonitrile and ether solvents. As used herein, the term "ether solvents" encompasses linear, branched and cyclic monoethers and linear, branched and cyclic polyethers. Preferably, the ether solvent is selected from THF, diethyl ether, *t*-butylmethyl ether, dibutylether, cyclopropylmethylether, 2-methyl-THF, 1,4-dioxane and polyethers of the structure R⁷(OR⁸)ₛOR⁹, wherein R⁷ and R⁹ are independently selected from a C₁₋₆ alkyl group, R⁸ is selected from C₂₋₄ alkylene, and s is selected from 1 to 5. More preferably, the ether solvent is selected from THF, diethyl ether, *t*-butylmethyl ether, dibutylether, cyclopropylmethylether, 2-methyl-THF, 1,4-dioxane, and diglyme.

More preferably the solvent S2 is selected from THF, diethyl ether, *t*-butylmethyl ether, dibutylether, cyclopropylmethylether, 2-methyl-THF, and 1,4-dioxane. Even more preferably, the solvent S2 is THF.

It will be understood that, typically, all solvents used in the composition in accordance with the invention are liquid at ambient conditions, e.g. 25°C and 1013 mbar. Moreover, it will be understood that any solvents which are present in the organozinc composition or which are used for their preparation are typically dry, i.e. essentially free or free of water, and of a purity sufficient for synthesis.

As will be understood from the above, in a preferred embodiment the composition in accordance with the invention comprises as component (c) a solvent mixture consisting of 1,3-dimethyl-3,4,5,6-tetrahydro-2(1*H*)-pyrimidinone or *N*,*N*,*N*',*N*'-tetramethylurea, more preferably 1,3-dimethyl-3,4,5,6-tetrahydro-2(1*H*)-pyrimidinone, as solvent S1 and an ether solvent selected from THF, diethyl ether, *t*-butylmethyl ether, dibutylether, cyclopropylmethylether, 2-methyl-THF, and 1,4-dioxane, more preferably THF, as solvent S2.

In a solvent mixture comprising at least one solvent S1 and at least one further solvent S2 different from S1, it is preferred that S1 is contained in the solvent mixture in an amount of 3 vol% or more, preferably 5 vol% or more, more preferably 10 vol% or more, based on the total volume of solvents contained in the composition.

At the same time, it is preferred for the solvent mixture comprising at least one solvent S1 and at least one further solvent S2 different from S1, that S1 is contained in the solvent mixture in an amount of 50 vol% or less, preferably 30 vol% or less, more preferably 20 vol% or less, based on the total volume of solvents contained in the composition. In such mixtures, the solvent S1 can also be referred to as a co-solvent.

Thus, it is especially preferred in the compositions in accordance with the invention to use, as component (c), a solvent mixture comprising 3 vol% to 50 vol%, more preferably 5 vol% to 30 vol%, and most preferably 10 vol% to 20 vol% of at least one solvent S1, the remainder, adding up to the 100 vol% as the total volume of solvents contained in the composition, being a solvent S2 as defined above.

As will be understood, the zinc complex and the lithium salt are typically dissolved in the solvent or solvent mixture. Preferably, the zinc complex and the lithium salt are dissolved in the solvent or solvent mixture such that the organozinc composition forms a single phase solution.

Preferably, the concentration of the zinc complex in the organozinc composition is from 0.10 to 1.10 mol/l, more preferably from 0.15 to 0.60 mol/l, even further preferably from 0.20 to 0.50 mol/l, and most preferably from 0.30 to 0.45 mol/l in terms of the molar amount of zinc contained in the organozinc composition.

### Organozinc composition

As set out above, the combination of the zinc complex (a), the lithium salt (b) and the solvent or solvent mixture (c) provides the advantageous characteristics of the organozinc compositions in accordance with the invention. It will be understood in this respect that the reference to a composition comprising components (a), (b) and (c) encompasses mixtures between the components, and also encompasses the case that two of the components (a), (b) and (c) or all three of them are interacting, e.g. that components (b) and/or (c) further coordinate with the zinc complex (a). Typically, the lithium cation Li⁺ of component and/or the anion X⁻ of component (b) is coordinated to the zinc complex of component (a).

The organozinc composition in accordance with the invention may comprise further components in addition to (a), (b) and (c). However, this is not necessary, such that the composition may also consist of components (a), (b) and (c). It is preferred that the composition is essentially free of magnesium, i.e. it does not contain magnesium, e.g. in the form of a magnesium compound such as a magnesium salt, in amounts which could have any influence on the reactivity of the composition. For example, "essentially free of magnesium" can refer to maximum amounts of 0.01 mol/l, preferably 0.001 mol/l, of magnesium (in terms of the molar amount of Mg in the organozinc composition). More preferably, the organozinc composition is free of magnesium.

The molar ratio of the zinc complex (a) to the lithium salt (b) (e.g. in terms of Zn to Li) in the organozinc composition in accordance with the invention preferably ranges from 1.0 : 1.0 to 1.0 : 3.0, more preferably from 1.0 : 1.5 to 1.0 : 2.5, and is most preferably 1.0 : 2.0.

In a particularly preferred embodiment, the zinc complex and the lithium salt form a compound of the following formula (Ia):

(R^{a}R^{b}N)₂Zn · 2LiX (Ia),

wherein R^{a}, R^{b} and X are defined as above, including all preferred embodiments.

The amount of the solvent or solvent mixture (c) is typically chosen such that a solution of components (a) and (b) is provided, preferably a single phase solution. More preferably, the amount of solvent or solvent mixture is selected such that a maximum concentration of the components (a) and (b) is contained in the solution.

As will be understood from the above, particularly preferred embodiments of the organozinc composition in accordance with the invention comprise
(a) an zinc complex, wherein a zinc atom carries two identical or different amide ligands of formula (I), which amide ligands are bound to the zinc atom via their nitrogen atom:

   R^{a}R^{b}N (I)

   wherein R^{a} and R^{b} are independently selected from cyclohexyl, isopropyl, trimethylsilyl, and *t*-butyl, and more preferably R^{a} and R^{b} are both cyclohexyl;
(b) a lithium salt of the formula LiX, wherein X is an anion selected from the group consisting of Cl⁻, Br⁻, I⁻, SCN⁻, CN⁻, NCO⁻, tosylate (p-tolylsulfonate) mesylate (methanesulfonate) and R'O⁻, wherein R' is C₁₋₄ alkyl, and more preferably X is Cl⁻;
(c) a solvent mixture comprising at least one solvent S1 selected from 1,3-dimethyl-3,4,5,6-tetrahydro-2(1*H*)-pyrimidinone and *N*,*N*,*N*',*N*'-tetramethylurea, more preferably 1,3-dimethyl-3,4,5,6-tetrahydro-2(1*H*)-pyrimidinone, and an ether solvent selected from THF, diethyl ether, *t*-butylmethyl ether, dibutylether, cyclopropylmethylether, 2-methyl-THF, and 1,4-dioxane, and more preferably THF, as further solvent S2.

An even further preferred embodiment of the invention in line with the above is an organozinc composition comprising a compound of the following formula (Ia):

(R^{a}R^{b}N)₂Zn · 2LiX (Ia),

wherein R^{a} and R^{b} are independently selected from cyclohexyl, isopropyl, trimethylsilyl, and *t-*butyl, and more preferably R^{a} and R^{b} are both cyclohexyl; and X is an anion selected from the group consisting of Cl⁻, Br⁻, I⁻, SCN⁻, CN⁻, NCO⁻, tosylate (p-tolylsulfonate) mesylate (methanesulfonate) and R'O⁻, wherein R' is C₁₋₄ alkyl, and more preferably X is Cl⁻; and a solvent mixture comprising at least one solvent S1 selected from 1,3-dimethyl-3,4,5,6-tetrahydro-2(1*H*)-pyrimidinone and *N*,*N*,*N*',*N*'-tetramethylurea, more preferably 1,3-dimethyl-3,4,5,6-tetrahydro-2(1*H*)-pyrimidinone, and an ether solvent selected from THF, diethyl ether, *t*-butylmethyl ether, dibutylether, cyclopropylmethylether, 2-methyl-THF, and 1,4-dioxane, and more preferably THF, as further solvent S2.

### Process for the preparation of the organozinc composition

A variety of synthetic approaches can be used for the preparation of the organozinc composition in accordance with the invention.

A first exemplary process for the preparation of the organozinc composition in accordance with the invention is a process comprising a step wherein an amine of the formula NHR^{a}R^{b}, wherein R^{a} and R^{b} are defined as above, is reacted with a base, preferably a lithium base, or with metallic lithium, to provide an amide R^{a}R^{b}N⁻, and a step wherein the amide is reacted with a Zn salt, preferably a Zn salt of the formula ZnX₂, wherein X is defined as above.

The solvent S1 or solvent mixture comprising at least one solvent S1 can be used as a reaction solvent, e.g. in the step reacting the amide with the Zn salt, or both in the step of providing the amide and in the step of reacting the amide with the Zn salt, or a reaction solvent other than S1 or solvent mixture comprising at least one solvent S1 is used and is replaced after the reaction by the solvent S1 or solvent mixture comprising at least one solvent S1.

A convenient and preferred process for the preparation of the organozinc composition in accordance with the invention in line with the above first exemplary process thus comprises the steps of:
(a) reacting an amine of the formula NHR^{a}R^{b}, wherein R^{a} and R^{b} are defined as above, including preferred embodiments,
   and a lithium base or metallic lithium to obtain an amide R^{a}R^{b}NLi; and
(b) reacting the amide with a Zn salt of the formula ZnX₂, wherein the anion X is defined as the anion X in component (b) of the composition in accordance with the invention,
   to obtain a zinc complex wherein two identical or different amide ligands of formula (I) as defined above are bound to a zinc atom, and a lithium salt of the formula LiX;
   wherein
   either at least the reaction of step b) is carried out in a solvent S1 or a solvent mixture comprising at least one solvent S1 as defined above;
   or wherein a solvent present in the reaction mixture obtained from reaction step b) is replaced by a solvent S1 or a solvent mixture comprising at least one solvent S1 as defined above.

Preferably, the reaction is carried out as a "one-pot" reaction starting from NHR^{a}R^{b}, the lithium base and ZnX₂ as reactants.

As will be understood from the above, during the process for the preparation of the organozinc composition in accordance with the invention and in line with the first exemplary process, the organozinc complex (a) is typically formed via a complexation of amide ligands to the zinc ion. The amide ligands can be obtained by deprotonation of the corresponding amine by a lithium base. The zinc salt, preferably ZnX₂, can be added either prior to, during or after reacting the amine and the lithium base. Preferably, it is added after the reaction of the amine and the lithium base. As will be understood, the anion of the zinc salt used in this process preferably corresponds to the anion which is contained in component (b) of the organozinc composition prepared by the process.

Suitable lithium bases include, for example, n-butyllithium, lithium *tert*-butoxide, Schlosser's base, cyclopentadienyllithium, LiH, and lithium hydroxide. Preferably the lithium base is an alkyllithium base or an alkoxylithium base; more preferably the lithium base is n-butyllithium.

As will be understood by the skilled reader, the zinc salt and the amide is typically used in a molar ratio, i.e. moles Zn/moles R^{a}R^{b}N⁻ or preferably moles ZnX₂/moles R^{a}R^{b}NLi, of about 0.50, such as 0.40 to 0.60, preferably 0.45 to 0.55, and most preferably 0.50.

Suitable reaction conditions for this process are e.g. analogous to those described for the preparation of Zn-Monoamide complexes in WO 2010/092096. For example, the amine and the lithium base can be reacted in a solvent, preferably a polar aprotic solvent such as THF. The reaction temperature is typically < 0°C, e.g. in the range of -50°C to -10°C. The reaction of the amide with the Zn salt is also typically carried out in a solvent, preferably a polar aprotic solvent such as THF. The reaction temperature is typically in the range of -50°C to room temperature (e.g. 25°C).

A second exemplary process for the preparation of the organozinc composition in accordance with the invention is a process comprising a step wherein an amine of the formula NXR^{a}R^{b}, wherein X, R^{a} and R^{b} are defined as above, is reacted with metallic zinc, preferably in the form of zinc powder, and step wherein LiX, wherein X is defined as above, is added. Preferably, the reaction of the amine with metallic zinc is carried out in the presence of LiX.

The solvent S1 or solvent mixture comprising at least one solvent S1 can be used as a reaction solvent, e.g. in the step reacting the amine with the metallic zinc, or the reaction solvent can be replaced after the reaction by the solvent S1 or solvent mixture comprising at least one solvent S1.

A convenient and preferred process for the preparation of the organozinc composition in accordance with the invention in line with the above second exemplary process thus comprises the steps of:
(a) providing an amine of the formula NXR^{a}R^{b}, wherein X, R^{a} and R^{b} are defined as above,
(b) reacting the amine of the formula NXR^{a}R^{b} with metallic zinc, preferably in the form of zinc powder,
(c) adding LiX prior to, during or after the reaction of step (b), wherein X is defined as above,
to obtain a composition comprising a zinc complex wherein a zinc atom carries two identical or different amide ligands of formula (I) as defined above, and a lithium salt of the formula LiX;
wherein
either at least the reaction of step (b) is carried out in a solvent S1 or a solvent mixture comprising at least one solvent S1 as defined above;
or wherein a solvent present in the reaction mixture obtained from reaction step (b) is replaced by a solvent S1 or a solvent mixture comprising at least one solvent S1 as defined above.

As noted above, the reaction of step (b) is preferably carried out in the presence of LiX.

The amine of the formula NXR^{a}R^{b} can be provided by methods known to the skilled practitioner. For example, in the preferred case where X is Cl⁻, an amine NHR^{a}R^{b} can be chlorinated with a chlorinating agent such as N-chloro-succinimide, or with a hypochlorite salt such as sodium hypochlorite.

As will be understood by the skilled reader, the zinc salt and the amide is typically used in a molar ratio, i.e. moles Zn/moles NXR^{a}R^{b} of about 0.50, such as 0.40 to 0.60, preferably 0.45 to 0.55, and most preferably 0.50.

The amount of LiX to be added is preferably such that the molar ratio of zinc to LiX (e.g. in terms of Zn to Li) ranges from 1.0 : 1.0 to 1.0 : 3.0, more preferably from 1.0 : 1.5 to 1.0 : 2.5, and is most preferably 1.0 : 2.0.

Suitable reaction conditions for this process are e.g. analogous to those described for the preparation of Zn-Monoamide complexes in A. Unsinn et al., Org. Lett., Vol. 15, No. 5, 2013, pages 1128-1131. For example, the amine and the metallic zinc can be reacted in a solvent, preferably a polar aprotic solvent such as THF. The reaction temperature is typically in the range of 40°C to -10°C, more preferably in the range of 25°C to 0°C.

Preferably, the presence of water is avoided as far as possible in the processes for the preparation of an organozinc composition in accordance with the invention. To that extent, it is preferred to adopt one or more of the following measures: carry out the process under protective gas, such as argon or nitrogen; use of dry solvents; and use of dry solid components.

### Synthetic procedures using the organozinc composition

The organozinc composition of the invention can be conveniently and efficiently used as a metalating reagent for the metalation of organic compounds, such as a wide variety of organic compounds comprising a Lewis acidic hydrogen atom. Thus, valuable tools for organic synthesis can be provided. It has been found that the metalation using the organozinc composition in accordance with the invention proceeds with a high selectivity, even for sensitive substrates, e.g. organic compounds carrying sensitive functional groups. The metalation reaction can be conveniently carried out at temperatures up to 100°C. Thus, the metalation reaction can be carried out safely, efficiently and also economically, and is scalable without problems.

In accordance with further aspects, the invention thus provides a process for the preparation of an organozinc compound of formula (IIIa) and/or (IIIb)

R^{c}-Zn-R^{c} (IIIa)

R^{c}-Zn-NR^{a}R^{b} (IIIb)

wherein
R^{c} is an organic group bound to the Zn atom via a carbon atom, and wherein the two groups R^{c} in formula (IIIa) are identical or different, preferably identical,
and R^{a} and R^{b} are defined as above,
said process comprising the step of reacting
(i) an organozinc composition in accordance with the invention with
(ii) an organic compound of the formula R^{c}-H, wherein R^{c} is defined as above, and wherein the hydrogen atom -H is bound to a carbon atom.

In the context of the present invention, the organic compound R^{c}-H may be referred to as a "substrate" in the process for the preparation of an organozinc compound.

Preferably, the organozinc compound prepared by the process is a compound of formula (IIIa), since the compound of formula (IIIa) provides two potentially reactive organic groups R^{c} per zinc atom.

It will be understood by the skilled reader that the -H in the organic compound of the formula R^{c}-H indicates a hydrogen atom that is replaced by Zn in the preparation of an organozinc compound of formula (IIIa) and/or (IIIb). Preferably, the hydrogen atom -H indicated for the compound of the formula R^{c}-H is a Lewis acidic hydrogen atom.

Preferred organic compounds R^{c}-H are those wherein R^{c} is selected from a substituted or unsubstituted aryl group, a substituted or unsubstituted heteroaryl group, a substituted or unsubstituted aralkyl group, preferably a benzyl group, a substituted or unsubstituted heteroaralkyl group, preferably a heterobenzyl group, a substituted or unsubstituted alkenyl group, and a substituted or unsubstituted alkynyl group, and from combinations of two or more of these groups, such as two, three or four thereof, wherein a first one of these groups forms a substituent for a second one of these groups, e.g. a substituted aryl group, which carries as one substituent a substituted or unsubstituted heteroaryl group.

As exemplary and preferred substituents for a cyclic organic group or a cyclic organic moiety of R^{c}, such as an aryl group, a heteroaryl group, the aryl moiety of an aralkyl group or the heteroaryl moiety of a heteroaralkyl group, reference can be made to halogen (i.e. F, Cl, Br, and I), -CN, C₁₋₆ alkyl, in particular methyl, C₁₋₆ fluoroalkyl, in particular trifluoromethyl, -NO₂,-NR^{x}R^{y}, -C(O)OR^{z}, -OR^{z}, C₁₋₆ alkenyloxy-C₁₋₆ alkoxy, -SR^{z}, -C(O)H, -C(O)R^{z}, wherein R^{x} and R^{y} are independently selected from H, C₁₋₆ alkyl, C₃₋₆ cycloalkyl, and phenyl, and R^{z} is selected from C₁₋₆ alkyl, C₃₋₆ cycloalkyl, and phenyl. As exemplary and preferred substituents for a non-cyclic organic group or a non-cyclic organic moiety, such as an alkenyl group, an alkynyl group, the alkylene moiety of an aralkyl group, or the alkylene moiety of a heteroaralkyl group, reference can be made to halogen (i.e. F, Cl, Br, and I), -CN, -NO₂, -NR^{x}R^{y}, -C(O)OR^{z}, -OR^{z}, C₁₋₆ alkenyloxy-C₁₋₆ alkoxy, -SR^{z}, -C(O)H, -C(O)R^{z}, wherein R^{x} and R^{y} are independently selected from H, C₁₋₆ alkyl, C₃₋₆ cycloalkyl, and phenyl, and R^{z} is selected from C₁₋₆ alkyl, C₃₋₆ cycloalkyl, and phenyl.

Among the more preferred organic compounds R^{c}-H are those wherein R^{c} is selected from a substituted aryl group, preferably a phenyl group, and a substituted heteroaryl group, preferably a 5 or 6-membered heteroaryl group containing one or more, preferably 1 or 2, heteroatoms selected from N, S and O. In these groups, the hydrogen atom indicated in the formula R^{c}-H is bound directly to a ring member of the aryl or heteroaryl group, typically a ring member which is a carbon atom. Preferably, the substituted aryl group and the substituted heteroaryl group comprise 1 to 3 substituents. Preferably the substituents are selected from halogen (i.e. F, Cl, Br, and I, more preferably F, Cl and Br), -CN, -NO₂,-C(O)OR^{z}, -OR^{z}, C₁₋₆ alkenyloxy-C₁₋₆ alkoxy, -SR^{z}, -C(O)H, -C(O)R^{z}, wherein R^{z} is selected from C₁₋₆ alkyl, C₃₋₆ cycloalkyl, and phenyl.

In further preferred embodiments of the process for the preparation of an organozinc compound of formula (IIIa) and/or (IIIb), R^{c} is selected from
(i) an organic group comprising an aryl or heteroaryl moiety which is bound to the Zn atom via a carbon atom contained as a ring member in the aryl or heteroaryl moiety, and wherein the hydrogen atom -H in the compound of formula R^{c}-H is bound to a carbon atom contained as a ring member in the aryl or heteroaryl moiety;
(ii) an organic group comprising a benzyl or heterobenzyl moiety which is bound to the Zn atom via a methylene group attached to an aryl or heteroaryl group, and wherein the hydrogen atom -H in the compound of formula R^{c}-H is bound to a methylene group attached to an aryl or heteroaryl group;
(iii) an organic group comprising a carbonyl group (-C(O)-), in particular a ketone group or an ester group, which organic group is bound to the Zn atom via a carbon atom in α-position relative to the carbonyl group, and wherein the hydrogen atom -H in the compound of formula R^{c}-H is bound to a carbon atom in α-position relative to a carbonyl group;
(iv) an organic group comprising a C-C double bond or triple bond which is bound to the Zn atom via a carbon atom involved in the C-C double bond or triple bond, and wherein the hydrogen atom -H in the compound of formula R^{c}-H is bound to a carbon atom involved in the C-C double bond or triple bond.

In a preferred exemplary embodiment of option (i) above, the group R^{c} comprises a heteroaryl ring containing two nitrogen atoms as ring members in adjacent positions.

As noted above, the organozinc composition in accordance with the invention allows the metalation of substrates carrying a sensitive functional group while maintaining the structural integrity of the sensitive functional group, the compound R^{c}-H and the group R^{c}, respectively, including the preferred embodiments discussed above, may carry one or more sensitive functional groups e.g. selected from halogen (i.e. F, Cl, Br, and I, more preferably F, Cl and Br), -CN, -NO₂, -C(O)OR^{z}, -OR^{z}, C₁₋₆ alkenyloxy-C₁₋₆ alkoxy, -SR^{z}, -C(O)H, -C(O)R^{z}, wherein R^{z} is selected from C₁₋₆ alkyl, C₃₋₆ cycloalkyl, and phenyl.

The reaction is typically carried out in a solvent, preferably a polar aprotic solvent. For example, the solvent may be an ether solvent, including linear, branched or cyclic monoethers and linear, branched or cyclic polyethers. Preferably the ether solvent is selected from THF, diethyl ether, *t*-butylmethyl ether, dibutylether, cyclopropylmethylether, 2-methyl-THF, 1,4-dioxane and polyethers of the structure R⁷(OR⁸)ₛOR⁹, wherein R⁷ and R⁹ are independently selected from a C₁₋₆ alkyl group, R⁸ is selected from C₂₋₄ alkylene, and s is selected from 1 to 5, more preferably from THF, diethyl ether, *t*-butylmethyl ether, dibutylether, cyclopropylmethylether, 2-methyl-THF, 1,4-dioxane, and diglyme.

The reaction is typically carried out at temperatures in the range of 0 to 100 °C.

The reaction of the organozinc composition with the compound of the formula R^{c}-H can be carried out e.g. in a batch reactor or in a flow reactor, and is preferably carried out in a flow reactor, more preferably under conditions of a continuous flow.

As will be understood by the skilled person, "continuous flow" typically refers to the case that reactants are continuously charged and/or mixed and may react continuously, without substantial, preferably without any interruption. The reactants can be charged all at once or subsequently. As explained below, additional reactants can be added in the course of the reaction. This means that a process carried out under continuous flow conditions can be carried out without any significant interruption of the reaction and/or without changing the basic setup. However, it is also conceivable that small discrete volume segments are continuously provided. Such a "quasi-continuous" provision of volume segments is also referred to as continuous flow in the context of the present invention. "Without substantial interruption" means that any possible interruptions are so minor that the reaction may proceed without being disturbed and/or that no substantial changes in the reaction sequence are necessary.

A continuous flow can be provided via methods known to the skilled person. For example, a peristaltic pump or an HPLC-pump can be used. Since their working cycles are very fast, the resulting flow can be considered to be continuous. Also the fast addition of small discrete volume segments, as used in capillary batch injection analysis (CBIA), is applicable and considered in the context of the present invention is a continuous flow.

In a preferred embodiment of a reaction using continuous flow conditions, the reaction solutions are pumped themselves with appropriate concentrations of the reactants.

In another preferred embodiment, a continuous solvent flow is established to which defined volumes or amounts of the reactants are added. Also this variant is referred to as continuous flow in the context of the present invention.

The flow reactor can be of any type established for continuous reactions. For example, a suitable micro reactor allows an excellent mixing of the reactants and/or dissipation of the reaction heat due to its dimensions and due to the high ratio of surface to volume. A "microreactor" is typically a reactor with a small diameter, e.g. in the range of up to 5 mm, preferably 2 mm, for example 0.8 to 1 mm.

Advantages of continuous flow conditions or a continuous flow reaction system may include, for example, faster mixing of the reagents, faster homogenization of the reaction solution and/or faster dissipation of heat, as well as the possibility of up- or downscaling the reaction volumes without problems in particular, it has been found that the use of a flow reactor allows an upscaling without further optimization, since the relationship between the reactants remains unchanged in the flow reaction. Both an arithmetic as well as a geometric upscaling is suitable.

However, depending on the circumstances, for example the amount and the nature of the reactants, fast or slow flow rates can be used. The skilled person will be in a position to adapt the flow rates appropriately. Preferred flow rates are such that the reaction rate of the desired reaction is large enough under the chosen conditions that the reactants can react sufficiently quickly and to a large degree. "Sufficiently quickly" means in this context that the portion of reactant added per time unit can react before the next portion of reactant arrives, such that no accumulation of not reacted reactants can occur. The appropriate flow rate is thus the function of the reaction conditions and the reaction rate(s).

In a preferred embodiment, at least two continuous streams of reactants are combined or mixed, i.e. two continuously processed substance streams are combined. For example, a continuously processed stream comprising the composition in accordance with the invention is combined or mixed with a further continuously processed stream comprising at least one compound R^{c}-H. For example, R^{c}-H can be dissolved in a solvent S2 as defined above.

Preferably, the presence of water should be avoided as far as possible in the process for the preparation of an organozinc compound in accordance with the invention. To that extent, it is preferred to adopt one or more of the following measures: carry out the process under protective gas, such as argon or nitrogen; use of dry solvents; and use of dry solid components.

It will be understood that the organozinc compound of formula (IIIa) and/or (IIIb) may be isolated, or may be used as a reactant in further reactions without being isolated, so that it exists only as an intermediate in a reaction sequence.

Yet a further aspect of the invention relates to a coupling process comprising the steps of:
(i) preparing an organozinc compound of formula (IIIa) and/or (IIIb), preferably (IIIa), in accordance with the process defined above; and
(ii) reacting the organozinc compound of formula (IIIa) and/or (IIIb), preferably (IIIa), with an electrophil E or a reactant comprising an electrophilic moiety E.

As will be understood by the skilled reader, a coupling process as referred to herein is typically a process wherein a covalent bond is formed between two chemical moieties, e.g. between two organic groups, or between an organic group and a halide. In the present case, such a bond is formed in the coupling process in particular between a group R^{c} of the organozinc compound of formula (IIIa) and/or (IIIb), and the electrophil E or the electrophilic moiety E of the reactant containing an electrophilic moiety.

Preferably, the reaction of step (ii) between the organozinc compound of formula (IIIa) and/or (IIIb) with an electrophil E or a reactant comprising an electrophilic moiety E is carried out in the presence of a transition metal catalyst, more preferably a transition metal catalyst comprising Pd and/or Cu; and even more preferably the transition catalyst comprises Pd. However, the reaction of step (ii) can also be carried out in the absence of any catalyst.

As will be understood by the skilled reader, an electrophil E or a reactant comprising an electrophilic moiety E is a compound which is at least partially formed of C- and H-Atoms and comprises an electron-poor site which may react with another molecule comprising a site with an increased electron density. The "electrophilic moiety" or an "electrophil" typically carries a positive charge or a positive partial charge.

In principle, various types of electrophils E or reactants comprising an electrophilic moiety E can be used, e.g. as disclosed in
- Handbook of Grignard reagents, edited by Gary S. Silverman and Philip E. Rakita (Chemical industries; v.64);
- Grignard reagents New Developments; edited by Herman g. Richey, Jr., 2000, John Wiley & Sons Ltd;
- Methoden der Organischen Chemie, Houben-Weyl, Band XIII/2a, Metallorganische Verbindungen Be, Mg, Ca, Sr, Ba, Zn, Cd, 1973;
- The chemistry of the metal-carbon bond, vol. 4, edited by Frank R. Hartley, 1987, John Wiley & Sons.

In particular, as an electrophil E or a reactant containing an electrophilic moiety E, an Lewis-acidic organic or inorganic reactant with an electron poor site can be used.

As will be understood by the skilled person, the electrophil E or the electrophilic moiety E will be able, due to its electrophilic properties, to replace the Zn atom in the bond formed between R^{c} and Zn in the organozinc compound of formula (IIIa) and/or (IIIb), and to form a bond with R^{c} so as to yield an organic compound R^{c}-E. As will also be understood, the electrophilic properties of the electrophil E or the electrophilic moiety E are not necessarily retained, and will typically not be present or not be present to the same extent in the organic compound R^{c}-E as they were present in the electrophil E or the reactant comprising the electrophilic moiety E.

One exemplary type of a reactant containing an electrophilic moiety E is an organic compound containing a leaving group, i.e. a group that departs with a pair of electrons in heterolytic bond cleavage in the reaction between the organozinc compound of formula (IIIa) and/or (IIIb) and the reactant containing an electrophilic moiety E. Exemplary leaving groups can be selected from Cl, Br and I, preferably Br and I, methanesulfonate (mesylate), and p-toluenesulfonate (tosylate).

For example, the reactant containing an electrophilic moiety E can be selected from aryl halides, in particular iodides or bromides, heteroaryl halides, in particular iodides or bromides, allylic halides, in particular allylic bromides, and halogens, like iodine (I₂) or bromine (Br₂). The allylic halides include cyclic and acyclic allylic halides. The aryl halogenides, heteroaryl halogenides and allylic halides may contain one or more, such as one, two or three halide substituents. Optionally, they may contain further substituents apart from the halide substituents.

As exemplary and preferred further substituents for the aryl halide or a heteroaryl halide, reference can be made to -OH, -CN, C₁₋₆ alkyl, in particular methyl, C₁₋₆ fluoroalkyl, in particular trifluoromethyl, -NO₂, -NR^{x}R^{y}, -C(O)OR^{z}, -OR^{z}, C₁₋₆ alkenyloxy-C₁₋₆ alkoxy, -SH,-SR^{z}, -C(O)H, -C(O)R^{z}, wherein R^{x} and R^{y} are independently selected from H, C₁₋₆ alkyl, C₃₋₆ cycloalkyl, and phenyl, and R^{z} is selected from C₁₋₆ alkyl, C₃₋₆ cycloalkyl, and phenyl. As exemplary and preferred substituents for the allylic halide, reference can be made to -OH, - CN, -NO₂, -NR^{x}R^{y}, -C(O)OR^{z}, -OR^{z}, C₁₋₆ alkenyloxy-C₁₋₆ alkoxy, -SH, -SR^{z}, -C(O)H, -C(O)R^{z}, wherein R^{x} and R^{y} are independently selected from H, C₁₋₆ alkyl, C₃₋₆ cycloalkyl, and phenyl, and R^{z} is selected from C₁₋₆alkyl, C₃₋₆ cycloalkyl, and phenyl.

The reaction of the organozinc compound of formula (IIIa) and/or (IIIb), preferably (IIIa), with an electrophil E or a reactant containing an electrophilic moiety E in step (ii) is typically carried out in a solvent, preferably a polar aprotic solvent. For example, the solvent may be an ether solvent, including linear, branched or cyclic monoethers and linear, branched or cyclic polyethers. Preferably the ether solvent is selected from THF, diethyl ether, *t*-butylmethyl ether, dibutylether, cyclopropylmethylether, 2-methyl-THF, 1,4-dioxane and polyethers of the structure R⁷(OR⁸)ₛOR⁹, wherein R⁷ and R⁹ are independently selected from a C₁₋₆ alkyl group, R⁸ is selected from C₂₋₄ alkylene, and s is selected from 1 to 5, more preferably from THF, diethyl ether, *t*-butylmethyl ether, dibutylether, cyclopropylmethylether, 2-methyl-THF, 1,4-dioxane, and diglyme.

The reaction of the organozinc compound of formula (IIIa) and/or (IIIb), preferably (IIIa), with an electrophil or a reactant containing an electrophilic moiety E in step (ii) can be carried out e.g. at temperatures in the range of 0 to 100 °C.

The reaction of the organozinc compound of formula (IIIa) and/or (IIIb), preferably (IIIa), with an electrophil or a reactant containing an electrophilic moiety E in step (ii) can be carried out e.g. in a batch reactor or in a flow reactor.

It will be understood that the organozinc compound of formula (IIIa) and/or (IIIb) prepared in step (i) may be isolated prior to step (ii), or may be reacted in step (ii) without being isolated prior to step (ii). It is preferred that the organozinc compound of formula (IIIa) and/or (IIIb) prepared in step (i) is reacted in step (ii) without being isolated prior to step (ii).

Still a further aspect of the invention relates to a process for the formation of an organometallic compound comprising a group R^{c} bound to a metal atom M, wherein R^{c} is defined as above, and M is a metal or semi-metal other than Zn, said process comprising the steps of:
(i) preparing an organozinc compound of formula (IIIa) and/or (IIIb) in accordance with the process for the preparation of an organozinc compound of formula (IIIa) and/or (IIIb), preferably (IIIa), as defined above; and
(ii) reacting the organozinc compound of formula (IIIa) and/or (IIIb) in a transmetalation reaction with M or a salt or an organometallic compound comprising M.

M is preferably boron or a metal which is more noble than Zn, i.e. a metal which acts as an electron acceptor in relation to Zn. Particularly preferred metals M are Cu or In.

Preferably, the organozinc compound of formula (IIIa) and/or (IIIb) is reacted in step (ii) with a metal salt, e.g. a halogenide or a cyanide, with a boron trihalogenide or with an organoboron compound. As preferred organoboron compounds, trialkylborates and bispinacol borane may be mentioned.

Thus, in a preferred embodiment of the above process for the formation of an organometallic compound, the reaction of the organozinc compound of formula (IIIa) and/or (IIIb) in a transmetalation reaction takes place with a Cu salt, preferably a Cu(I) salt, in particular CuCN, CuCI or CuBr, or with an indium salt, in particular an indium trihalogenide.

The transmetalation reaction in step (ii) is typically carried out in a solvent, preferably a polar aprotic solvent. For example, the solvent may be an ether solvent, including linear, branched or cyclic monoethers and linear, branched or cyclic polyethers. Preferably the ether solvent is selected from THF, diethyl ether, *t*-butylmethyl ether, dibutylether, cyclopropylmethylether, 2-methyl-THF, 1,4-dioxane and polyethers of the structure R⁷(OR⁸)ₛOR⁹, wherein R⁷ and R⁹ are independently selected from a C₁₋₆ alkyl group, R⁸ is selected from C₂₋₄ alkylene, and s is selected from 1 to 5, more preferably from THF, diethyl ether, *t*-butylmethyl ether, dibutylether, cyclopropylmethylether, 2-methyl-THF, 1,4-dioxane, and diglyme.

The transmetalation reaction in step (ii) can be carried out e.g. at temperatures in the range of 0 to 100 °C.

The transmetalation reaction in step (ii) can be carried out e.g. in a batch reactor or in a flow reactor.

It will be understood that the organozinc compound of formula (IIIa) and/or (IIIb) prepared in step (i) may be isolated prior to step (ii), or may be reacted in step (ii) without being isolated prior to step (ii). It is preferred that the organozinc compound of formula (IIIa) and/or (IIIb) prepared in step (i) is reacted in step (ii) without being isolated prior to step (ii).

### General Definitions

As used herein, "alkyl" generally refers to a linear or branched chain saturated hydrocarbon residue which does not comprise any carbon-to-carbon double bonds or carbon-to-carbon triple bonds. As exemplary groups, methyl, ethyl, propyl and butyl are mentioned. The term "alkylene" refers to a divalent linear or branched chain saturated hydrocarbon residue which does not comprise any carbon-to-carbon double bonds or carbon-to-carbon triple bonds.

As used herein, "alkenyl" generally refers to a straight or branched chain unsaturated hydrocarbon residue comprising one or more than one (such as two or three) carbon-to-carbon double bond(s) which does not comprise any carbon-to-carbon triple bonds.

As used herein, "alkynyl" generally refers to a straight or branched chain unsaturated hydrocarbon residue comprising one or more than one (such as two or three) carbon-to-carbon triple bond(s). It will be understood that an "alkynyl" may also comprise one or more than one (such as two or three) carbon-to-carbon double bonds.

As used herein, "aryl" generally refers to an aromatic hydrocarbon ring, in particular a 6 to 10 membered ring (unless a different number of ring members is indicated in a specific context), including bridged ring or fused ring systems containing at least one aromatic ring. Preferred as aryl groups are monocyclic groups with 6 ring members or fused bicyclic groups with 9 or 10 ring members. Thus, generally preferred embodiments of "aryl" are phenyl or naphthyl. Particularly preferred is a phenyl group.

As used herein, "aralkyl" generally refers to an alkylene group, carrying an aryl group at one of its valencies. A benzyl group represents a preferred embodiment of an aralkyl group, wherein the alkylene moiety is a methylene group and the aryl group is a phenyl group.

As used herein, "heteroaryl" generally refers to an aromatic ring, preferably a 5-14 membered ring (unless a different number of ring members is indicated in a specific context), including bridged ring or fused ring systems containing at least one aromatic ring, comprising one or more (such as, e.g., one, two, or three) heteroatoms as ring members of the aromatic ring. Preferably, the heteroatoms are independently selected from O, S, and N. More preferred as heteroaryl groups are monocyclic groups with 5 or 6 members and fused bicyclic groups with 8 to 10 ring members. Particularly preferred as heteroaryl groups are monocyclic groups with 5 or 6 members. "Heteroaryl" may, for example, refer to thienyl (thiophenyl), benzo[b]thienyl, naphtho[2,3-b]thienyl, thianthrenyl, furyl (furanyl), isobenzofuranyl, chromenyl, xanthenyl, phenoxathiinyl, pyrrolyl (including, without limitation, 2H-pyrrolyl), imidazolyl, pyrazolyl, pyridyl (pyridinyl; including, without limitation, 2-pyridyl, 3-pyridyl, and 4-pyridyl), pyrazinyl, pyrimidinyl, pyridazinyl, indolizinyl, isoindolyl, indolyl (including, without limitation, 3H-indolyl), indazolyl, purinyl, isoquinolyl, quinolyl, phthalazinyl, naphthyridinyl, quinoxalinyl, cinnolinyl, pteridinyl, carbazolyl, beta-carbolinyl, phenanthridinyl, acridinyl, phenanthrolinyl (including, without limitation, [1,10]phenanthrolinyl, [1,7]phenanthro-linyl, and [4,7]phenanthrolinyl), phenazinyl, isothiazolyl, phenothiazinyl, oxazolyl, isoxazolyl, furazanyl, phenoxazinyl, pyrazolo[1,5-a]pyrimidinyl (including, without limitation, pyrazolo[1,5-a]pyrimidin-3-yl), 1,2-benzoisoxazol-3-yl, or benzimidazolyl.

As used herein, "heteroaralkyl" generally refers to an alkylene group, carrying a heteroaryl group at one of its valencies. A heterobenzyl group represents a preferred embodiment of heteroaralkyl group, wherein the alkylene moiety is a methylene group and the aryl group is a 6-membered heterocyclic group.

As used herein, "cycloalkyl" generally refers to a saturated hydrocarbon ring, preferably a 3-8 membered ring (unless a different number of ring members is indicated in a specific context), including bridged ring, spiro ring or fused ring systems. "Cycloalkyl" may, for example, refer to cyclopropyl, cyclobutyl, cyclopentyl, cyclohexyl, or cycloheptyl. Preferred as cycloalkyl groups are monocyclic groups with 5 or 6 ring members.

Various groups are referred to as being "optionally substituted" or "substituted or unsubstituted" in the context of this description. Unless indicated otherwise, these groups may carry one or more than one, such as e.g. one, two, three or four substituents. It will be understood that the maximum number of substituents is limited by the number of attachment sites available on the substituted moiety. Unless defined otherwise in the specific context, these groups carry preferably not more than two substituents and may, in particular, carry only one substituent. As exemplary and preferred substituents for a cyclic organic group or a cyclic organic moiety, such as an aryl group, a heteroaryl group, a cycloalkyl group, the aryl moiety of an aralkyl group or the heteroaryl moiety of a heteroaralkyl group, reference can be made to halogen (i.e. F, Cl, Br, and I), -OH, -CN, C₁₋₆ alkyl, in particular methyl, C₁₋₆ fluoroalkyl, in particular trifluoromethyl, -NO₂, -NR^{x}R^{y}, -C(O)OR^{z}, --OR^{z}, C₁₋₆ alkenyloxy-C₁₋₆ alkoxy, -SH, -SR^{z}, -C(O)H, -C(O)R^{z}, wherein R^{x} and R^{y} are independently selected from H, C₁₋₆ alkyl, C₃₋₆ cycloalkyl, and phenyl, and R^{z} is selected from C₁₋₆ alkyl, C₃₋₆ cycloalkyl, and phenyl. As exemplary and preferred substituents for a non-cyclic organic group or a non-cyclic organic moiety, such as an alkyl group, an alkenyl group, an alkynyl group, the alkylene moiety of an aralkyl group, or the alkylene moiety of a heteroaralkyl group, reference can be made to halogen (i.e. F, Cl, Br, and I), -OH, -CN, -NO₂, -NR^{x}R^{y}, -C(O)OR^{z},-OR^{z}, C₁₋₆ alkenyloxy-C₁₋₆ alkoxy, -SH, -SR^{z}, -C(O)H, -C(O)R^{z}, wherein R^{x} and R^{y} are independently selected from H, C₁₋₆ alkyl, C₃₋₆ cycloalkyl, and phenyl, and R^{z} is selected from C₁₋₆ alkyl, C₃₋₆ cycloalkyl, and phenyl. However, unless specifically defined otherwise, it is preferred that the substituents are absent. This applies irrespective of whether reference is made to an "optionally substituted" group, or a group which may be "substituted or unsubstituted".

Important aspects of the present invention are summarized in the following items
1. An organozinc composition comprising:
   (a) a zinc complex, wherein a zinc atom carries two identical or different amide ligands of formula (I), which amide ligands are bound to the zinc atom via their nitrogen atom :

      R^{a}R^{b}N (I)

      wherein R^{a} and R^{b} are independently selected from a saturated C₁₋₁₀ hydrocarbon group,
      and wherein optionally
      one or more of the carbon atoms in the hydrocarbon group may be replaced by a silicon atom each;
      a group R^{a} and a group R^{b} may be connected in the same ligand R^{a}R^{b}N to form a 5 to 8 membered ring together with the nitrogen atom to which they are attached; and/or
      a group R^{a} or R^{b} of one amide ligand may be connected with a group R^{a} or R^{b} of the other amide ligand, such that a bidentate ligand comprising two amide groups is formed;
   (b) a lithium salt of the formula LiX,
      wherein
      X is an anion selected from the group consisting of H⁻, F⁻, Cl⁻, Br⁻, I⁻, SCN⁻, CN⁻, NCO⁻, HalOₘ⁻, wherein Hal is selected from Cl, Br, and I and m is 3 or 4, NOₚ⁻, wherein p is 2 or 3,BF₄⁻, PF₆⁻, R'O⁻, R'C(O)O⁻, R'S⁻, R'C(O)S⁻, R'C(S)O⁻, R'C(S)S⁻, R'R"N⁻, R'(R"O)P(O)O⁻, R'O(R"O)P(O)O⁻, R'R"P⁻, R'SO₃⁻, R'SO₂S⁻, and R'OSO₃⁻, wherein R' is H or an organic group selected from aryl, heteroaryl, alkaryl, alkyl, cycloalkyl, alkenyl and alkynyl, which organic group may be optionally substituted, wherein R" is H or an organic group selected from aryl, heteroaryl, alkaryl, alkyl, cycloalkyl, alkenyl and alkynyl, which organic group may be optionally substituted, and wherein two suitable organic groups R' and R" may be connected to form a ring together with the atoms to which they are attached;
   (c) a solvent S1 or a solvent mixture comprising at least one solvent S1, wherein S1 is a solvent of formula (II) : 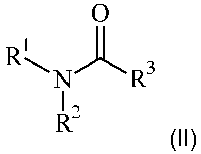 wherein
      R¹ is selected from H and a saturated C₁₋₁₀ hydrocarbon group;
      R² is a saturated C₁₋₁₀ hydrocarbon group;
      and R³ is selected from NR⁴R⁵, H and R⁶, wherein R⁴ is selected from H and a saturated C₁₋₁₀ hydrocarbon group, R⁵ is a saturated C₁₋₁₀ hydrocarbon group and R⁶ is a saturated C₁₋₁₀ hydrocarbon group;
      and wherein optionally
      R² and R⁵ or R² and R⁶ may be connected to form a 5 to 8 membered ring together with the atoms to which they are attached.
2. The organozinc composition according to item 1, wherein the lithium cation Li⁺ and/or the anion X⁻ of component (b) is coordinated to the zinc complex of component (a).
3. The organozinc composition according item 1 or 2, wherein the zinc complex and the lithium salt form a compound of the following formula (Ia):

   (R^{a}R^{b}N)₂Zn · 2LiX (Ia),

   wherein R^{a}, R^{b} and X are defined as in item 1.
4. The organozinc composition according to any of items 1 to 3, wherein R^{a} and R^{b} are independently selected from a linear or branched C₁₋₁₀ alkyl group, a C₃₋₈ cycloalkyl group, a C₃₋₁₀ trialkylsilyl group, and a structure wherein a group R^{a} and a group R^{b} are connected in the same ligand R^{a}R^{b}N form a 2,2,6,6-tetramethylpiperidyl ring together with the nitrogen atom to which they are attached.
5. The organozinc composition according to any of items 1 to 3, wherein at least one of R^{a} and R^{b} in each amide ligand, preferably R^{a} and R^{b}, are independently selected from a branched C₃₋₇ alkyl group, a C₅₋₇ cycloalkyl group, and a C₃₋₁₀ trialkylsilyl group.
6. The organozinc composition according to any of items 1 to 3, wherein R^{a} and R^{b} are independently selected from cyclohexyl, isopropyl, trimethylsilyl, and *t*-butyl.
7. The organozinc composition according to any of items 1 to 3, wherein R^{a} and R^{b} are both cyclohexyl.
8. The organozinc composition according to any of items 1 to 7, wherein X is selected from Cl⁻, Br⁻, I⁻, SCN⁻, CN⁻, NCO⁻, tosylate, mesylate, and R'O⁻, wherein R' is C₁₋₄ alkyl.
9. The organozinc composition according to any of items 1 to 7, wherein X is Cl⁻.
10. The organozinc composition according to any of items 1 to 9, wherein S1 is a solvent of formula (IIa) or (IIb), preferably (IIa): 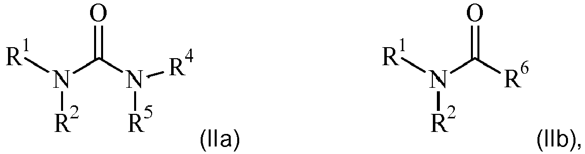 wherein
   R¹ is selected from H and a saturated C₁₋₁₀ hydrocarbon group, preferably from H and a linear or branched C₁₋₁₀ alkyl group, and more preferably from H, methyl and ethyl;
   R² is a saturated C₁₋₁₀ hydrocarbon group, preferably a linear or branched C₁₋₁₀ alkyl group;
   R⁴ is selected from H, and a saturated C₁₋₁₀ hydrocarbon group, preferably from H and a linear or branched C₁₋₁₀ alkyl group, and more preferably from H, methyl and ethyl;
   R⁵ is a saturated C₁₋₁₀ hydrocarbon group, preferably a linear or branched C₁₋₁₀ alkyl group;
   and R⁶ is selected from H and a saturated C₁₋₁₀ hydrocarbon group, preferably from H and a linear or branched C₁₋₁₀ alkyl group;
   and wherein optionally
   R² and R⁵ or R² and R⁶ may be connected to form a 5 to 8 membered ring together with the atoms to which they are attached.
11. The organozinc composition according to item 10, wherein R¹ is selected from H, methyl and ethyl, R² is a linear or branched C₁₋₆ alkyl group, R⁴ is selected from H, methyl and ethyl, R⁵ is a linear or branched C₁₋₆ alkyl group, R⁶ is selected from H and a linear or branched C₁₋₆ alkyl group; and wherein optionally R² and R⁵ or R² and R⁶ may be connected to form a 5 or 6 membered ring together with the atoms to which they are attached.
12. The organozinc composition according any of items 1 to 9, wherein S1 is selected from the group consisting of 1,3-dimethyl-3,4,5,6-tetrahydro-2(1*H*)-pyrimidinone (DMPU), *N,N,N*'*,N*'-tetramethylurea (TMU), *N*-methyl-2-pyrrolidone (NMP), *N*-ethyl-2-pyrrolidone (NEP), and dimethylformamide (DMF).
13. The organozinc composition according any of items 1 to 9, wherein S1 is selected from the group consisting of 1,3-dimethyl-3,4,5,6-tetrahydro-2(1*H*)-pyrimidinone and *N*,*N*,*N*',*N*'-tetramethylurea, and is more preferably 1,3-dimethyl-3,4,5,6-tetrahydro-2(1*H*)-pyrimidinone.
14. The organozinc composition according any of items 1 to 13, which comprises a solvent S1 as the only solvent, or a solvent mixture comprising at least one solvent S1 and a further solvent S2 different from S1.
15. The organozinc composition according to item 14, which comprises a solvent mixture comprising at least one solvent S1 and a further solvent S2 different from S1, wherein S1 is contained in the solvent mixture in an amount of 3 vol% or more, preferably 5 vol% or more, more preferably 10 vol% or more, based on the total volume of solvents contained in the composition.
16. The organozinc composition according to item 14 or 15, which comprises a solvent mixture comprising at least one solvent S1 and a further solvent S2 different from S1, wherein S1 is contained in the solvent mixture in an amount of 50 vol% or less, preferably 30 vol% or less, more preferably 20 vol% or less, based on the total volume of solvents contained in the composition.
17. The organozinc composition according to any of items 14 to 16, wherein the solvent S2 is selected from the group consisting of acetonitrile and ether solvents including linear, branched or cyclic monoethers and linear, branched or cyclic polyethers, preferably from THF, diethyl ether, *t*-butylmethyl ether, dibutylether, cyclopropylmethylether, 2-methyl-THF, 1,4-dioxane and polyethers of the structure R⁷(OR⁸)ₛOR⁹, wherein R⁷ and R⁹ are independently selected from a C₁₋₆ alkyl group, R⁸ is selected from C₂₋₄ alkylene, and s is selected from 1 to 5, more preferably from THF, diethyl ether, *t*-butylmethyl ether, dibutylether, cyclopropylmethylether, 2-methyl-THF, 1,4-dioxane, and diglyme.
18. The organozinc composition of any of items 1 to 17, wherein the zinc complex and the lithium salt are dissolved in the solvent or solvent mixture.
19. The organozinc composition of any of items 1 to 18, wherein the concentration of the zinc complex in the organozinc composition is from 0.10 to 1.10 mol/l, preferably from 0.15 to 0.60 mol/l, and more preferably from 0.20 to 0.50 mol/l, in terms of the molar amount of zinc contained in the organozinc composition.
20. A process for the preparation of the organozinc composition as defined in any one of items 1 to 19, said process comprising the steps of :
   (a) reacting an amine of the formula NHR^{a}R^{b}, wherein R^{a} and R^{b} are defined as in the preceding items,
      and a lithium base or metallic lithium to obtain an amide R^{a}R^{b}NLi; and
   (b) reacting the amide with a Zn salt of the formula ZnX₂, wherein X is defined as in the preceding items
      to obtain a composition comprising a zinc complex wherein a zinc atom carries two identical or different amide ligands of formula (I) as defined in the preceding items, and a lithium salt of the formula LiX;
      wherein
      either at least the reaction of step b) is carried out in a solvent S1 or a solvent mixture comprising at least one solvent S1 as defined in the preceding items;
      or wherein a solvent present in the reaction mixture obtained from reaction step b) is replaced by a solvent S1 or a solvent mixture comprising at least one solvent S1 as defined in the preceding items.
21. A process for the preparation of the organozinc composition as defined in any one of items 1 to 19, said process comprising the steps of :
   (a) providing an amine of the formula NXR^{a}R^{b}, wherein X, R^{a} and R^{b} are defined as in the preceding items,
   (b) reacting the amine of the formula NXR^{a}R^{b} with metallic zinc, preferably in the form of zinc powder,
   (c) adding LiX prior to, during or after the reaction of step (b), wherein X is defined as in the preceding items,
   to obtain a composition comprising a zinc complex wherein a zinc atom carries two identical or different amide ligands of formula (I) as defined in the preceding items, and a lithium salt of the formula LiX;
   wherein
   either at least the reaction of step (b) is carried out in a solvent S1 or a solvent mixture comprising at least one solvent S1 as defined in the preceding items;
   or wherein a solvent present in the reaction mixture obtained from reaction step (b) is replaced by a solvent S1 or a solvent mixture comprising at least one solvent S1 as defined in the preceding items.
22. The process of claim 21, wherein the reaction of step (b) is carried out in the presence of the LiX.
23. A process for the preparation of an organozinc compound of formula (IIIa) and/or (IIIb), preferably (IIIa),

   R^{c}-Zn-R^{c} (IIIa)

   R^{c}-Zn-NR^{a}R^{b} (IIIb)

   wherein
   R^{c} is an organic group bound to the Zn atom via a carbon atom, and wherein the two groups R^{c} in formula (IIIa) are identical or different, preferably identical,
   and R^{a} and R^{b} are defined as in the preceding items,
   said process comprising the step of reacting
   (i) an organozinc composition of any of items 1 to 19 with
   (ii) an organic compound of the formula R^{c}-H, wherein R^{c} is an organic group and wherein the hydrogen atom -H is bound to a carbon atom.
24. The process in accordance with item 23, wherein the reaction of the organozinc composition of any of items 1 to 19 with the compound of the formula R^{c}-H is carried out in a flow reactor, preferably under conditions of a continuous flow.
25. The process in accordance with item 23 or 24, wherein R^{c} in formula (IIIa) or (IIIb) is selected from
   (i) an organic group comprising an aryl or heteroaryl moiety which is bound to the Zn atom via a carbon atom contained as a ring member in the aryl or heteroaryl moiety, and wherein the hydrogen atom -H in the compound of formula R⁷-H is bound to a carbon atom contained as a ring member in the aryl or heteroaryl moiety;
   (ii) an organic group comprising a benzyl or heterobenzyl moiety which is bound to the Zn atom via a methylene group attached to an aryl or heteroaryl group, and wherein the hydrogen atom -H in the compound of formula R^{c}-H is bound to a methylene group attached to an aryl or heteroaryl group;
   (iii) an organic group comprising a carbonyl group (-C(O)-), in particular a ketone group or an ester group, which is bound to the Zn atom via a carbon atom in α-position relative to the carbonyl group, and wherein the hydrogen atom -H in the compound of formula R^{c}-H is bound to a carbon atom in α-positionrelative to a carbonyl group; and
   (iv) an organic group comprising a C-C double bond or triple bond which organic group is bound to the Zn atom via a carbon atom involved in the C-C double bond or triple bond, and wherein the hydrogen atom -H in the compound of formula R^{c}-H is bound to a carbon atom involved in the C-C double bond or triple bond.
26. The process in accordance with any of items 23 to 25, wherein the group R^{c} further comprises one or more substituents selected from an ester group, -CN and -NO₂.
27. The process in accordance with any of items 23 to 26, wherein the group R^{c} is an organic group comprising a heteroaryl moiety, and the comprises a heteroaryl moiety contains two nitrogen atoms as ring members in adjacent positions.
28. A coupling process comprising the steps of:
   (i) preparing an organozinc compound of formula (IIIa) and/or (IIIb), preferably (IIIa), in accordance with the process of any of items 23 to 27; and
   (ii) reacting the organozinc compound of formula (IIIa) and/or (IIIb), preferably (IIIa), with an electrophil E or a reactant comprising an electrophilic moiety E.
29. The process of item 28, wherein the reaction of step (ii) is carried out in the presence of a transition metal catalyst.
30. A process for the formation of an organometallic compound comprising a group R^{c} bound to a metal atom M, wherein R^{c} is defined as in items 23 to 27 and M is a metal or semi-metal other than Zn, said process comprising the steps of:
   (i) preparing an organozinc compound of formula (IIIa) and/or (IIIb), preferably (IIIa), in accordance with the process of any of items 23 to 27; and
   (ii) reacting the organozinc compound of formula (IIIa) and/or (IIIb), preferably (IIIa), in a transmetalation reaction with a M or a metal salt or an organometallic compound comprising M.
31. The process of item 30, wherein M is Cu, and wherein, in step (ii), the organozinc compound of formula (IIIa) and/or (IIIb), preferably (IIIa), is reacted with a Cu(I) salt as a metal salt.
31. Use of the organozinc composition as defined in any of items 1 to 19 as a metalation reagent for the metalation of an organic compound.

### Examples

### General considerations

Reactions were carried out with a FlowSyn system purchased from Uniqsis. Metalations were performed in a coiled reactor and a tube (1.0 mm id; 20 and 0.4 mL) made from PFA, PTFE Teflon or stainless steel. Carrier solvents as well as reactant solutions were stored under argon. Reactions with electrophiles were carried out under batch conditions with magnetic stirring and in flame-dried glassware under argon. Syringes, which were used to transfer reagents and solvents, were purged with argon prior to use.

### Solvents

Solvents were dried according to standard methods by distillation from drying agents as stated below and were stored under argon. Otherwise they were obtained from commercial sources and used without further purification.

THF was continuously refluxed and freshly distilled from sodium benzophenone ketyl under nitrogen.

DMPU was heated to reflux for 14 h over CaH₂ and distilled from CaH₂.

Solvents for column chromatography were distilled prior to use.

### Reagents

All reagents were obtained from commercial sources and used without further purification unless otherwise stated.

**Cy₂NH** was distilled prior to use and stored under argon.

**n-BuLi** solution in hexane was purchased from Rockwood Lithium and the concentration was determined by titration against 1,10-phenanthroline in THF with *i*-PrOH.

**CuCN·2LiCl** solution (1.00 M in THF) was prepared by drying CuCN (8.96 g; 100 mmol) and LiCl (8.48 g; 200 mmol) in a Schlenk flask under vacuum for 5 h at 150 °C. After cooling to 25 °C, dry THF (100 mL) was added and stirred until the salts were dissolved.

**ZnCl₂** solution (1.00 M in THF) was prepared by drying ZnCl₂ (27.3 g; 200 mmol) in a Schlenk flask under vacuum for 5 h at 150 °C. After cooling to 25 °C, dry THF (200 mL) was added and stirred until the salt was dissolved.

**(Cy₂N)₂Zn·2LiCl** (solution in THF/DMPU = 10:1) was prepared by slow addition of n-BuLi (32.8 mL; 80 mmol; 2.5 M in hexane) to a solution of Cy₂NH (14.8 g; 16.4 mL; 80 mmol) in THF (80 mL) at -40 °C and slowly warmed to -20 °C within 30 min. ZnCl₂ (40 mL; 40 mmol; 1.00 M in THF) was added at -20 °C and the solution was slowly warmed to 25 °C within 2 h. All solvents were removed *in vacuo,* the remaining residue was dissolved in freshly distilled THF and 10 vol% DMPU and stirred until a clear solution was obtained. The concentration was determined by titration against benzoic acid in THF using 4-(phenylazo)diphenylamine as indicator.

### Chromatography

Flash column chromatography was performed using SiO₂ 60 (0.040-0.063 mm, 230-400 mesh ASTM) from Merck.

Thin layer chromatography (TLC) was performed using aluminium plates covered with SiO₂ (Merck 60, F-254). Spots were visualized under UV light and/or by staining of the TLC plate with one of the solutions below, followed by heating with a heat gun:
- KMnO₄ (0.3 g), K₂CO₃ (20 g) and KOH (0.3 g) in water (300 mL).
- Neat iodine absorbed on silica gel.
- Phosphor molybdic acid (5.0 g), Ce(SO₄)₂ (2.0 g) and conc. H₂SO₄ (12.0 mL) in water (230 mL).

### Analytical data

NMR spectra were recorded on *Bruker* ARX 200, AC 300, WH 400 or AMX 600 instruments. Chemical shifts are reported as δ-values in ppm relative to the deuterated solvent peak: CDCl₃ (δ_{H}: 7.26; δ_{C}: 77.16). For the observation of the observed signal multiplicities, the following abbreviations were used: s (singlet), d (doublet), dd (doublet of doublets), t (triplet), q (quartet) and m (multiplet). If not otherwise noted, the coupling constants given are C-H-coupling constants. Melting points are uncorrected and were measured on a *Büchi* B.540 apparatus. Infrared spectra were recorded from 4000-400 cm⁻¹ on a *Nicolet* 510 FT-IR or a *Perkin-Elmer* 281 IR spectrometer. Samples were measured neat (*Smiths Detection* DuraSampl *IR* II Diamond ATR). The absorption bands are reported in wavenumbers (cm⁻¹). Gas chromatography (GC) was performed with instruments of the type *Hewlett-Packard* 6890 or 5890 Series II, using a column of the type HP 5 (*Hewlett-Packard*, 5% phenylmethylpolysiloxane; length: 10 m, diameter: 0.25 mm, film thickness: 0.25 µm). The detection was accomplished using a flame ionization detector. Mass spectra (MS) and high resolution mass spectra (HRMS) were recorded on a *Finnigan* MAT95Q or *Finnigan* MAT90 instrument for electron impact ionization (EI) and electrospray ionization (ESI). For the combination of gas chromatography with mass spectroscopic detection, a GC-MS of the type *Hewlett-Packard* 6890 / MSD 5793 networking was used (column: HP 5-MS, *Hewlett-Packard*; 5% phenylmethylpolysiloxane; length: 15 m, diameter 0.25 mm; film thickness: 0.25 µm).

### Typical procedures (TP)

### Typical procedure for the zincation in flow using (Cy₂N)₂Zn·2LiCl followed by the reaction with an electrophile in batch (TP1):

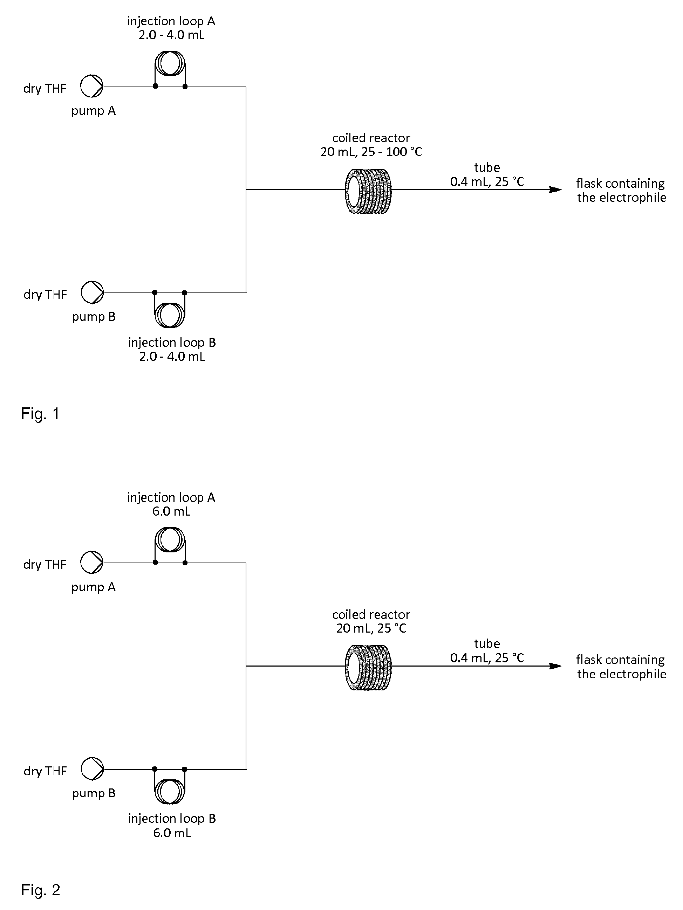

The flow system (FlowSyn, Uniqsis; schematically shown in **Fig. 1**) was dried by flushing it with dry THF (flow rate of all pumps: 1.00 mL/min; run-time: 30 min). Injection loop A (2.0 or 4.0 mL) was loaded with the reactant solution (0.36 - 0.78 M in dry THF; 3.0 or 5.0 mL) and injection loop B (2.0 or 4.0 mL) was loaded with (Cy₂N)₂Zn·2LiCl (0.20 - 0.43 M in dry THF + 10 vol% dry DMPU; 0.55 equiv; 3.0 or 5.0 mL). The solutions were simultaneously injected into separate THF streams (pump A and B, flow rates: 1.00 mL/min) respectively, before they were mixed in a coiled reactor followed by a tube (20.4 mL in total; residence time: 10 min; 25 - 100 °C). The combined streams were collected in a flame-dried, argon flushed 25 mL flask equipped with a magnetic stirrer and a septum containing the electrophile (0.8 equiv) dissolved in dry THF (2 mL). Then the reaction mixture was further stirred for the indicated time at the indicated temperature. The completion of the reaction was checked by GC analysis of reaction aliquots quenched with concentrated aqueous NH₄Cl solution and using undecane as an internal standard.

### Typical procedure for the scale-up zincation in flow using (Cy₂N)₂Zn·2LiCl followed by the reaction with an electrophile in batch (TP2):

The flow system (FlowSyn, Uniqsis; schematically shown in **Fig. 2**) was dried by flushing it with dry THF (flow rate of all pumps: 1.00 mL/min; run-time: 30 min). Injection loop A (6.0 mL) was loaded with the reactant solution (0.49 M in dry THF; 7.0 mL) and injection loop B (6.0 mL) was loaded with (Cy₂N)₂Zn·2LiCl (0.27 M in dry THF + 10 vol% dry DMPU; 0.55 equiv; 7.0 mL). The solutions were simultaneously injected into separate THF streams (pump A and B, flow rates: 1.00 mL/min) respectively, before they were mixed in a coiled reactor followed by a tube (20.4 mL in total; residence time: 10 min; 25 °C). The combined streams were collected in a flame-dried, argon flushed 250 mL flask equipped with a magnetic stirrer and a septum containing the electrophile (0.8 equiv) dissolved in dry THF (20 mL). After 7 min, the injection loops were reloaded with the reactant solution and (Cy₂N)₂Zn·2LiCl, injected into the separate THF streams again and collected in the same flask as well. The number of reloads was depending on the desired reaction scale. Then the reaction mixture was further stirred for the indicated time at the indicated temperature. The completion of the reaction was checked by GC analysis of reaction aliquots quenched with concentrated aqueous NH₄Cl solution and using undecane as an internal standard.

### Preparation of products

### Synthesis of ethyl 4-(3,6-dichloropyridazin-4-yl)benzoate

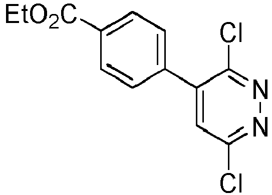

According to **TP1**, injection loop A and B (2.0 mL) were loaded with solutions of 3,6-dichloropyridazine (3.0 mL; 0.67 M) and (Cy₂N)₂Zn·2LiCl (3.0 mL; 0.37 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 4-iodobenzoate (295 mg; 1.07 mmol; 0.8 equiv), Pd(dba)₂ (15 mg; 2 mol%) and tfp (12 mg; 4 mol%) dissolved in THF (2 mL). The reaction mixture was further stirred for 2 h at 25 °C before it was quenched with water (30 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 7:1 + 0.5 vol% NEt₃) afforded the target compound as a yellow solid (229 mg; 0.77 mmol; 72%).
m.p. (°C): 94.5 - 96.3.
**¹H-NMR** (400 MHz, CDCl₃): δ / ppm = 8.11 (d, *J* = 8.4 Hz, 2H), 7.53 (d, *J* = 8.5 Hz, 2H), 7.50 (s, 1 H), 4.35 (q, *J* = 7.1 Hz, 2H), 1.35 (t, *J* = 7.1 Hz, 3H).
**¹³C-NMR** (101 MHz, CDCl₃): δ / ppm = 165.6, 156.1, 154.4, 141.8, 137.2, 132.1, 130.0, 129.6, 129.0, 61.5, 14.3.
**IR** (Diamond-ATR, neat): *ṽ* / cm⁻¹ = 3048, 2980, 2905, 2872, 2244, 1713, 1612, 1561, 1508, 1476, 1464, 1446, 1407, 1367, 1352, 1327, 1313, 1271, 1183, 1135, 1101, 1056, 1040, 1018,908,860,840,774,752,730,703.
**MS** (EI, 70 eV): m/z (%) = 298 (37), 297 (M⁺, 15), 296 (48), 270 (28), 268 (49), 254 (13), 253 (62), 252 (14), 251 (100), 188 (11), 160 (13), 153 (22), 126 (18), 99 (10).
**HRMS** (EI): calcd. for [C₁₃H₁₀Cl₂N₂O₂₁: 296.0119; found: 296.0116 (M⁺).

### Synthesis of 3,6-dichloro-4-(4-(trifluoromethyl)phenyl)pyridazine

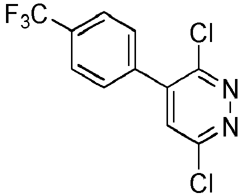

According to **TP1**, injection loop A and B (2.0 mL) were loaded with solutions of 3,6-dichloropyridazine (3.0 mL; 0.67 M) and (Cy₂N)₂Zn·2LiCl (3.0 mL; 0.37 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing 4-iodobenzotrifluoride (291 mg; 1.07 mmol; 0.8 equiv), Pd(dba)₂ (15 mg; 2 mol%) and tfp (12 mg; 4 mol%) dissolved in THF (2 mL). The reaction mixture was further stirred overnight at 25 °C before it was quenched with water (30 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 10:1 + 0.5 vol% NEt₃) afforded the target compound as a colorless solid (202 mg; 0.69 mmol; 64%).
**m.p.** (°C): 117.7 - 119.3.
**¹H-NMR** (400 MHz, CDCl₃): δ / ppm = 7.79 (d, *J* = 8.1 Hz, 2H), 7.61 (d, *J* = 8.1 Hz, 2H), 7.49 (s, 1H).
**¹³C-NMR** (101 MHz, CDCl₃): δ / ppm = 156.2, 154.4, 141.3, 136.7, 132.4 (q, *J* = 33.2 Hz), 129.6, 129.4, 126.0 (q, *J* = 4.0 Hz), 123.6 (q, *J* = 272.6 Hz).
**IR** (Diamond-ATR, neat): *ṽ* / cm⁻¹ = 3108, 3062, 3033, 2927, 2855, 1619, 1555, 1492, 1459, 1409, 1380, 1351, 1324, 1284, 1234, 1216, 1193, 1172, 1145, 1136, 1120, 1106, 1071, 1055, 1040, 1014, 975, 961, 920, 852, 845, 836, 773, 760, 741, 706, 658.
**MS** (EI, 70 eV): m/z (%) = 294 (24), 292 (38), 266 (58), 265 (11), 264 (78), 231 (20), 229 (66), 216 (18), 214 (28), 209 (14), 206 (30), 204 (100), 195 (17), 194 (50), 193 (19), 176 (11), 175 (11), 170 (25), 169 (39), 154 (28), 145 (18), 144 (21), 143 (10), 126 (12), 125 (19), 120 (10), 99 (22), 95 (16), 75 (22), 74 (13).
**HRMS** (EI): calcd. for [C₁₁H₅Cl₂F₃N₂]: 291.9782; found: 291.9763 (M⁺).

### Synthesis of 3,6-dichloro-4-(cyclohex-2-enyl)pyridazine

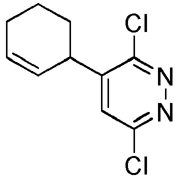

According to **TP1**, injection loop A and B (2.0 mL) were loaded with solutions of 3,6-dichloropyridazine (3.0 mL; 0.67 M) and (Cy₂N)₂Zn·2LiCl (3.0 mL; 0.37 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing 3-bromocyclohexene (172 mg; 1.07 mmol; 0.8 equiv) and CuCN·2LiCl (0.13 mL; 10 mol%) dissolved in THF (2 mL) and cooled to 0 °C. The reaction mixture was stirred for further 1 h at 0 °C and 30 min at 25 °C before it was quenched with sat. aq. NH₄Cl/NH₃ (10 vol%; 20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 30:1 + 0.5 vol% NEt₃) afforded the target compound as a colorless oil (211 mg; 0.92 mmol; 86%).
**¹H-NMR** (400 MHz, CDCl₃): δ / ppm = 7.34 (s, 1H), 6.12 - 6.07 (m, 1H), 5.55 - 5.50 (m, 1H), 3.72 - 3.68 (m, 1 H), 2.11 - 2.07 (m, 3H), 1.67 - 1.44 (m, 3H).
**¹³C-NMR** (101 MHz, CDCl₃): δ / ppm = 156.6, 156.1, 147.7, 132.6, 128.8, 124.8, 37.4, 28.4, 24.7, 19.7.
**IR** (Diamond-ATR, neat): *ṽ* / cm⁻¹ = 3025, 2930, 2862, 2836, 1651, 1559, 1509, 1446, 1431, 1393, 1356, 1342, 1312, 1293, 1275, 1252, 1227, 1212, 1191, 1127, 1100, 1058, 1047, 1038, 995, 921, 910, 880, 863, 851, 814, 764, 754, 723, 686, 666.
**MS** (EI, 70 eV): m/z (%) = 230 (24), 228 (35), 193 (10), 157 (24), 142 (21), 139 (17), 137 (55), 129 (20), 111 (23), 104 (14), 103 (12), 102 (35), 101 (14), 89 (13), 81 (100), 79 (14), 77 (32), 75 (19), 67 (41), 65 (12), 63 (17), 54 (24), 53 (23), 51 (40), 50 (16), 41 (42).
**HRMS** (EI): calcd. for [C₁₀H₁₀Cl₂N₂]: 228.0221; found: 228.0203 (M⁺).

### Synthesis of ethyl 2',6'-difluoro-3'-nitrobiphenyl-4-carboxylate

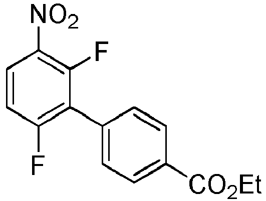

According to **TP1**, injection loop A and B (2.0 mL) were loaded with solutions of 2,4-difluoronitrobenzene (3.0 mL; 0.67 M) and (Cy₂N)₂Zn·2LiCl (3.0 mL; 0.37 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing ethyl 4-iodobenzoate (295 mg; 1.07 mmol; 0.8 equiv), Pd(dba)₂ (15 mg; 2 mol%) and tfp (12 mg; 4 mol%) dissolved in THF (2 mL). The reaction mixture was further stirred overnight at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 30:1) afforded the target compound as an orange solid (315 mg; 1.03 mmol; 96%).
**m.p.** (°C): 85.0 - 86.9.
**¹H-NMR** (400 MHz, CDCl₃): δ / ppm = 8.15 (d, *J* = 8.4 Hz, 2H), 8.14 - 8.09 (m, 1 H), 7.51 (d, *J* = 8.3 Hz, 2H), 7.16 - 7.11 (m, 1 H), 4.40 (q, *J* = 7.1 Hz, 2H), 1.40 (t, *J* = 7.1 Hz, 3H).
**¹³C-NMR** (101 MHz, CDCl₃): δ / ppm = 165.9, 162.5 (dd, *J* = 260.4 Hz, *J* = 6.0 Hz), 153.8 (dd, *J* = 267.5 Hz, *J* = 8.0 Hz), 134.7, 131.3, 130.2 (t, *J* = 2.0 Hz), 129.7, 126.6 (dd, *J* = 11.1 Hz, *J* = 2.0 Hz), 120.3 (dd, *J* = 20.1 Hz, *J* = 18.1 Hz), 112.2 (dd, *J* = 24.1 Hz, *J* = 4.0 Hz), 61.3, 14.3. (One signal not observed; possible coincidental isochronicity.)
**IR** (Diamond-ATR, neat): ν̃ / cm⁻¹ = 3101, 2984, 2904, 1891, 1712, 1619, 1589, 1567, 1536, 1510, 1472, 1405, 1367, 1342, 1304, 1286, 1269, 1214, 1184, 1148, 1127, 1103, 1070, 1021, 1010, 948, 879, 857, 824, 777, 757, 713, 702, 667.
**MS** (EI, 70 eV): m/z (%) = 307 (M⁺, 24), 279 (41), 263 (16), 262 (100), 216 (32), 188 (32), 187 (10).
**HRMS** (EI): calcd. for [C₁₅H₁₁F₂NO₄]: 307.0656; found: 307.0643 (M⁺).

### Scale-up synthesis of ethyl 2',6'-difluoro-3'-nitrobiphenyl-4-carboxylate

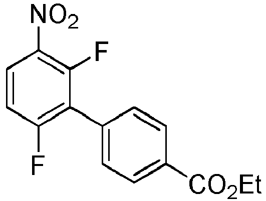

According to **TP2**, injection loop A and B (6.0 mL) were loaded with solutions of 2,4-difluoronitrobenzene (7.0 mL; 0.49 M) and (Cy₂N)₂Zn·2LiCl (7.0 mL; 0.27 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing ethyl 4-iodobenzoate (3.26 g; 11.8 mmol; 0.8 equiv), Pd(dba)₂ (169 mg; 2 mol%) and tfp (137 mg; 4 mol%) dissolved in THF (20 mL). The reaction loops were reloaded four times and the reaction mixture was further stirred overnight at 25 °C before it was quenched with water (150 mL). The aq. layer was extracted with EtOAc (3×200 mL), the combined organic fractions were dried over anhydrous Na₂SO₄, filtrated and the solvent was removed *in vacuo.* Purification by flash column chromatography (silica gel; *i-*hexane:EtOAc = 30:1) afforded the target compound as an orange solid (3.56 g; 11.6 mmol; 98%).

### Synthesis of 2,6-difluoro-4'-methoxy-3-nitrobiphenyl

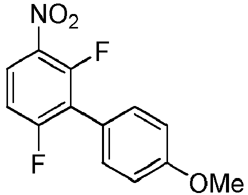

According to **TP1**, injection loop A and B (2.0 mL) were loaded with solutions of 2,4-difluoronitrobenzene (3.0 mL; 0.67 M) and (Cy₂N)₂Zn·2LiCl (3.0 mL; 0.37 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing 4-iodoanisole (250 mg; 1.07 mmol; 0.8 equiv), Pd(dba)₂ (15 mg; 2 mol%) and tfp (12 mg; 4 mol%) dissolved in THF (2 mL). The reaction mixture was further stirred overnight at 25 °C before it was quenched with water (30 mL). The aq. layer was extracted with EtOAc (3×80 mL), the combined organic fractions were dried over anhydrous Na₂SO₄, filtrated and the solvent was removed *in vacuo.* Purification by flash column chromatography (silica gel; *i*-hexane:EtOAc = 30:1) afforded the target compound as a yellow solid (216 mg; 0.81 mmol; 76%).
**m.p.** (°C): 104.5 - 106.8.
**¹H-NMR** (400 MHz, CDCl₃): δ / ppm = 8.06 - 8.00 (m, 1 H), 7.37 (d, *J* = 8.9 Hz, 2H), 7.11 - 7.06 (m, 1H), 7.00 (d, *J* = 8.9 Hz, 2H), 3.85 (s, 3H).
**¹³C-NMR** (101 MHz, CDCl₃): δ / ppm = 162.8 (dd, *J* = 258.5 Hz, *J* = 7.0 Hz), 160.3, 153.9 (dd, *J* = 265.5 Hz, *J* = 8.0 Hz), 134.9, 131.5 (t, *J* = 2.0 Hz), 125.5 (dd, *J* = 11.1 Hz, *J* = 1.0 Hz), 121.0 (dd, *J* = 20.1 Hz, *J* = 18.1 Hz), 118.9, 114.2, 112.0 (dd, *J* = 25.1 Hz, *J* = 4.0 Hz), 55.3.
**IR** (Diamond-ATR, neat): *ṽ* / cm⁻¹ = 3098, 2940, 2843, 1893, 1613, 1587, 1576, 1532, 1516, 1468, 1438, 1411, 1347, 1305, 1282, 1256, 1212, 1179, 1146, 1117, 1073, 1017, 1007, 954, 938, 876, 840, 822, 806, 765, 730, 689.
**MS** (EI, 70 eV): m/z (%) = 266 (14), 265 (M⁺, 100), 219 (38), 204 (21), 188 (21), 176 (39), 175 (48).
**HRMS** (EI): calcd. for [C₁₃H₉F₂NO₃]: 265.0551; found: 265.0542 (M⁺).

### Synthesis of ethyl 2-(2,6-difluoro-3-nitrobenzyl)acrylate

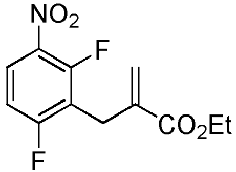

According to **TP1**, injection loop A and B (4.0 mL) were loaded with solutions of 2,4-difluoronitrobenzene (5.0 mL; 0.49 M) and (Cy₂N)₂Zn·2LiCl (5.0 mL; 0.27 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing ethyl 2-(bromomethyl)acrylate (303 mg; 1.57 mmol; 0.8 equiv) and CuCN·2LiCl (0.20 mL; 10 mol%) dissolved in THF (2 mL) and cooled to 0 °C. The reaction mixture was stirred for further 1 h at 0 °C and overnight at 25 °C before it was quenched with sat. aq. NH₄Cl/NH₃ (10 vol%; 20 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i-*hexane:EtOAc = 15:1) afforded the target compound as a yellow oil (301 mg; 1.11 mmol; 71%).
**¹H-NMR** (400 MHz, CDCl₃): δ / ppm = 8.04 - 7.98 (m, 1H), 7.03 - 6.98 (m, 1H), 6.28 (s, 1H), 5.43 (s, 1H), 4.19 (q, *J* = 7.1 Hz, 2H), 3.74 (s, 2H), 1.27 (t, *J* = 7.1 Hz, 3H).
**¹³C-NMR** (101 MHz, CDCl₃): δ/ppm= 165.9, 164.3 (dd, *J* = 259.5 Hz, *J* = 8.0 Hz), 155.3 (dd, *J* = 267.5 Hz, *J* = 9.1 Hz), 136.2, 134.4, 126.7, 125.7 (dd, *J* = 11.1 Hz, *J* = 1.0 Hz), 117.7 (dd, *J* = 20.1 Hz, *J* = 18.1 Hz), 111.6 (dd, *J* = 24.1 Hz, *J* = 4.0 Hz), 61.2, 25.2, 14.1.
**IR** (Diamond-ATR, neat): *ṽ* / cm⁻¹ = 3107, 2984, 2908, 2876, 1714, 1624, 1596, 1534, 1476, 1455, 1434, 1408, 1348, 1319, 1300, 1275, 1257, 1213, 1174, 1136, 1096, 1036, 951, 885, 859,823,765,739,715,680.
**MS** (EI, 70 eV): m/z (%) = 271 (M⁺, 23), 226 (58), 225 (52), 223 (15), 206 (12), 198 (12), 197 (40), 179 (15), 172 (13), 152 (21), 151 (100), 148 (23), 140 (10), 133 (12), 126 (14), 125 (14), 55(11).
**HRMS** (EI): calcd. for [C₁₂H₁₁F₂NO₄]: 271.0656; found: 271.0645 (M⁺).

### Synthesis of 4-bromo-3-(cyclohex-2-enyl)-2-fluorobenzonitrile

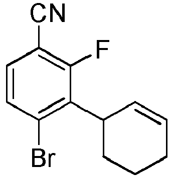

According to **TP1**, injection loop A and B (2.0 mL) were loaded with solutions of 4-bromo-2-fluorobenzonitrile (3.0 mL; 0.67 M) and (Cy₂N)₂Zn·2LiCl (3.0 mL; 0.37 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing 3-bromocyclohexene (172 mg; 1.07 mmol; 0.8 equiv) and CuCN·2LiCl (0.13 mL; 10 mol%) dissolved in THF (2 mL) and cooled to 0 °C. The reaction mixture was stirred for further 1 h at 0 °C and 30 min at 25 °C before it was quenched with sat. aq. NH₄Cl/NH₃ (10 vol%; 20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 30:1) afforded the target compound as a colorless oil (238 mg; 0.85 mmol; 79%).
**¹H-NMR** (400 MHz, CDCl₃): δ / ppm = 7.45 - 7.44 (m, 1H), 7.31 - 7.27 (m, 1H), 5.85 - 5.79 (m, 1H), 5.57 - 5.54 (m, 1H), 4.01 - 3.98 (m, 1H), 2.18 - 2.02 (m, 2H), 1.92 - 1.58 (m, 4H).
**¹³C-NMR** (101 MHz, CDCl₃): δ / ppm = 162.1 (d, *J* = 264.5 Hz), 134.8 (d, *J* = 13.1 Hz), 131.1, 131.1 (d, *J* = 1.0 Hz), 129.6 (d, *J* = 4.0 Hz), 127.8 (d, *J* = 2.0 Hz), 127.2 (d, *J* = 1.0 Hz), 113.6 (d, *J* = 1.0 Hz), 101.6 (d, *J* = 17.1 Hz), 27.7, 27.7, 24.4, 22.5.
**IR** (Diamond-ATR, neat): *ṽ* / cm⁻¹ = 3084, 3025, 2933, 2837, 2860, 2238, 1651, 1597, 1558, 1454, 1419, 1344, 1315, 1300, 1264, 1246, 1225, 1213, 1196, 1184, 1140, 1127, 1063, 1047, 1016, 946, 930, 898, 875, 858, 814, 754, 719, 644, 632, 612, 580, 557.
**MS** (EI, 70 eV): m/z (%) = 61 (13), 45 (13), 43 (100).
**HRMS** (EI): calcd. for [C₁₃H₁₁BrFN]: 279.0059; found: 279.0057 (M⁺).

### Synthesis of ethyl 6'-bromo-3'-cyano-2'-fluorobiphenyl-4-carboxylate

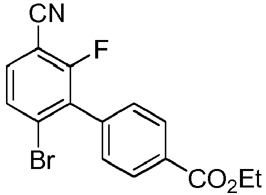

According to **TP1**, injection loop A and B (4.0 mL) were loaded with solutions of 4-bromo-2-fluorobenzonitrile (5.0 mL; 0.36 M) and (Cy₂N)₂Zn·2LiCl (5.0 mL; 0.20 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 4-iodobenzoate (317 mg; 1.15 mmol; 0.8 equiv) and Pd(PPh₃)₄ (83 mg; 5 mol%) dissolved in THF (2 mL). The reaction mixture was further stirred overnight at 25 °C before it was quenched with water (30 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 15:1) afforded the target compound as a yellow oil (310 mg; 0.89 mmol; 77%).
**¹H-NMR** (400 MHz, CDCl₃): δ / ppm = 8.15 (d, *J* = 8.6 Hz, 2H), 7.62 - 7.60 (m, 1 H), 7.51 - 7.48 (m, 1 H), 7.37 (d, *J* = 8.4 Hz, 2H), 4.40 (q, *J* = 7.1 Hz, 2H), 1.40 (t, *J* = 7.1 Hz, 3H).
**¹³C-NMR** (101 MHz, CDCl₃): δ / ppm = 166.0, 160.2 (d, *J* = 261.5 Hz), 136.8, 132.9 (d, *J* = 1.0 Hz), 131.7 (d, *J* = 17.1 Hz), 131.2, 130.3 (d, *J* = 3.0 Hz), 129.9 (d, *J* = 1.0 Hz), 129.8, 129.6 (d, *J* = 5.0 Hz), 113.2, 101.3 (d, *J* = 17.1 Hz), 61.3, 14.3.
**IR** (Diamond-ATR, neat): *ṽ* / cm⁻¹ = 3077, 2982, 2937, 2906, 2238, 1713, 1612, 1599, 1572, 1558, 1513, 1476, 1453, 1428, 1403, 1367, 1310, 1270, 1245, 1196, 1179, 1102, 1086, 1020, 964, 889, 854, 836, 817, 773, 751, 708, 696.
**MS** (EI, 70 eV): m/z (%) = 349 (26), 347 (25), 321 (53), 319 (54), 305 (20), 304 (99), 303 (19), 302 (100), 196 (16), 195 (95), 168 (13), 97 (19).
**HRMS** (EI): calcd. for [C₁₆H₁₁BrFNO₂]: 346.9957; found: 346.9960 (M⁺).

### Synthesis of 6-bromo-2-fluoro-4'-methoxybiphenyl-3-carbonitrile

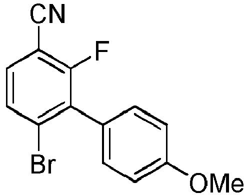

According to **TP1**, injection loop A and B (4.0 mL) were loaded with solutions of 4-bromo-2-fluorobenzonitrile (5.0 mL; 0.36 M) and (Cy₂N)₂Zn·2LiCl (5.0 mL; 0.20 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing 4-iodoanisole (269 mg; 1.15 mmol; 0.8 equiv) and Pd(PPh₃)₄ (83 mg; 5 mol%) dissolved in THF (2 mL). The reaction mixture was further stirred overnight at 25 °C before it was quenched with water (20 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i-*hexane:EtOAc = 20:1) afforded the target compound as a colorless solid (299 mg; 0.98 mmol; 85%).
**m.p.** (°C): 87.8 - 89.6.
**¹H-NMR** (400 MHz, CDCl₃): δ / ppm = 7.59 - 7.56 (m, 1 H), 7.44 - 7.41 (m, 1 H), 7.22 (d, *J* = 8.3 Hz, 2H), 6.99 (d, *J* = 8.5 Hz, 2H), 3.85 (s, 3H).
**¹³C-NMR** (101 MHz, CDCl₃): δ/ppm = 160.6 (d, *J =* 260.5 Hz), 160.1, 132.3 (d, *J* = 17.1 Hz), 132.1 (d, *J* = 1.0 Hz), 131.2 (d, *J* = 3.0 Hz), 131.0 (d, *J* = 2.0 Hz), 129.4 (d, *J* = 4.0 Hz), 124.5, 114.0, 113.6, 101.1 (d, *J* = 18.1 Hz), 55.3.
**IR** (Diamond-ATR, neat): *ṽ* / cm⁻¹ = 3071, 3004, 2949, 2844, 2240, 1904, 1722, 1666, 1642, 1609, 1597, 1578, 1564, 1554, 1514, 1471, 1451, 1440, 1423, 1406, 1300, 1278, 1242, 1180, 1132, 1115, 1088, 1062, 1028, 916, 887, 858, 834, 827, 816, 810, 782, 756, 723.
**MS** (EI, 70 eV): m/z (%) = 308 (17), 307 (100), 306 (M⁺, 16), 264 (19), 262 (21), 183 (28), 182 (26).
**HRMS** (EI): calcd. for [C₁₄H₉BrFNO]: 306.9831; found: 306.9816 (M⁺).

### Synthesis of ethyl 5-(4-(ethoxycarbonyl)phenyl)thiophene-2-carboxylate

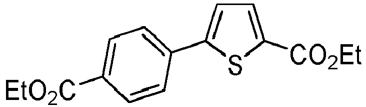

According to **TP1**, injection loop A and B (4.0 mL) were loaded with solutions of ethyl 2-thiophenecarboxylate (5.0 mL; 0.36 M) and (Cy₂N)₂Zn·2LiCl (5.0 mL; 0.20 M in THF + 10 vol% DMPU; 0.55 equiv). After injection (flow rate of pump A and B: 1.00 mL/min) and metalation (residence time: 10 min; metalation temperature: 100 °C) the combined streams were collected in a flask containing ethyl 4-iodobenzoate (317 mg; 1.15 mmol; 0.8 equiv), Pd(dba)₂ (17 mg; 2 mol%) and tfp (13 mg; 4 mol%) dissolved in THF (2 mL). The reaction mixture was further stirred overnight at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 30:1) afforded the target compound as a yellow solid (236 mg; 0.78 mmol; 68%).
**m.p.** (°C): 105.8 - 107.2.
**¹H-NMR** (400 MHz, CDCl₃): δ / ppm = 8.05 (d, *J* = 8.7 Hz, 2H), 7.75 (d, *J* = 3.9 Hz, 1H), 7.67 (d, *J* = 8.7 Hz, 2H), 7.35 (d, *J* = 3.9 Hz, 1H), 4.40 - 4.32 (m, 4H), 1.40 - 1.35 (m, 6H).
**¹³C-NMR** (101 MHz, CDCl₃): δ / ppm = 166.0, 162.1, 149.4, 137.5, 134.2, 133.9, 130.4, 130.4, 125.9, 124.8, 61.3, 61.2, 14.4, 14.3.
**IR** (Diamond-ATR, neat): *ṽ* / cm⁻¹ = 2987, 2976, 2928, 2905, 2870, 1699, 1605, 1566, 1536, 1507, 1476, 1449, 1410, 1388, 1362, 1342, 1314, 1288, 1268, 1250, 1212, 1184, 1112, 1095, 1052, 1012, 959, 904, 882, 856, 845, 818, 769, 746, 717, 694, 666.
**MS** (EI, 70 eV): m/z (%) = 305 (16), 304 (M⁺, 100), 276 (22), 260 (15), 259 (82), 233 (11), 232 (13), 158 (11), 115 (13).
**HRMS** (EI): calcd. for [C₁₆H₁₆O₄S]: 304.0769; found: 304.0759 (M⁺).

### Synthesis of ethyl 5-(3-methoxyphenyl)thiophene-2-carboxylate

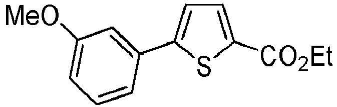

According to **TP1**, injection loop A and B (4.0 mL) were loaded with solutions of ethyl 2-thiophenecarboxylate (5.0 mL; 0.36 M) and (Cy₂N)₂Zn·2LiCl (5.0 mL; 0.20 M in THF + 10 vol% DMPU; 0.55 equiv). After injection (flow rate of pump A and B: 1.00 mL/min) and metalation (residence time: 10 min; metalation temperature: 100 °C) the combined streams were collected in a flask containing 3-iodoanisole (269 mg; 1.15 mmol; 0.8 equiv), Pd(dba)₂ (17 mg; 2 mol%) and tfp (13 mg; 4 mol%) dissolved in THF (2 mL). The reaction mixture was further stirred overnight at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 50:1) afforded the target compound as a yellow oil (193 mg; 0.74 mmol; 64%).
**¹H-NMR** (400 MHz, CDCl₃): δ / ppm = 7.72 (d, *J* = 3.9 Hz, 1H), 7.31 - 7.27 (m, 1H), 7.25 (d, *J* = 3.9 Hz, 1H), 7.21 - 7.19 (m, 1H), 7.14 - 7.13 (m, 1H), 6.89 - 6.86 (m, 1H), 4.34 (q, *J* = 7.1 Hz, 2H), 3.83 (s, 3H), 1.37 (t, *J* = 7.1 Hz, 3H).
**¹³C-NMR** (101 MHz, CDCl₃): δ / ppm = 162.3, 160.1, 150.9, 134.8, 134.1, 132.6, 130.1, 123.8, 118.7, 114.3, 111.8, 61.2, 55.3, 14.4.
**IR** (Diamond-ATR, neat): *ṽ* / cm⁻¹ = 3081, 2980, 2938, 2906, 2836, 1700, 1598, 1579, 1537, 1493, 1459, 1438, 1392, 1367, 1344, 1273, 1242, 1218, 1167, 1090, 1041, 1013, 994, 906, 864,839,814,774,746,684.
**MS** (EI, 70 eV): m/z (%) = 263 (18), 262 (M⁺, 100), 234 (45), 218 (15), 217 (80), 190 (17), 145 (23), 102 (11).
**HRMS** (EI): calcd. for [C₁₄H₁₄O₃S]: 262.0664; found: 262.0659 (M⁺).

### Synthesis of 5-(4-(trifluoromethyl)phenyl)furan-2-carbonitrile

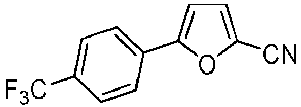

According to **TP1**, injection loop A and B (2.0 mL) were loaded with solutions of 2-furonitrile (3.0 mL; 0.69 M) and (Cy₂N)₂Zn·2LiCl (3.0 mL; 0.38 M in THF + 10 vol% DMPU; 0.55 equiv). After injection (flow rate of pump A and B: 1.00 mL/min) and metalation (residence time: 10 min; metalation temperature: 80 °C) the combined streams were collected in a flask containing 4-iodobenzotrifluoride (299 mg; 1.10 mmol; 0.8 equiv), Pd(dba)₂ (16 mg; 2 mol%) and tfp (13 mg; 4 mol%) dissolved in THF (2 mL). The reaction mixture was further stirred overnight at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 20:1) afforded the target compound as a yellow solid (167 mg; 0.70 mmol; 64%).
**m.p.** (°C): 89.3 - 90.9.
**¹H-NMR** (400 MHz, CDCl₃): δ / ppm = 7.81 (d, *J* = 8.2 Hz, 2H), 7.68 (d, *J* = 8.3 Hz, 2H), 7.18 (d, *J* = 3.7 Hz, 1H), 6.83 (d, *J* = 3.7 Hz, 1H).
**¹³C-NMR** (101 MHz, CDCl₃): δ / ppm = 156.9, 131.7, 131.2 (q, *J* = 33.2 Hz), 127.4, 126.1 (q, *J* = 4.0 Hz), 125.0, 123.9, 123.8 (q, *J* = 272.3 Hz), 111.5, 107.8.
**IR** (Diamond-ATR, neat): *ṽ* / cm⁻¹ = 3135, 2934, 2230, 1618, 1592, 1574, 1532, 1489, 1418, 1392, 1364, 1318, 1277, 1235, 1218, 1167, 1111, 1070, 1052, 1025, 967, 926, 895, 880, 846, 830, 797, 774, 741, 694, 670, 629, 593.
**MS** (EI, 70 eV): m/z (%) = 238 (11), 237 (M⁺, 100), 183 (26), 140 (22).
**HRMS** (EI): calcd. for [C₁₂H₆F₃NO]: 237.0401; found: 237.0397 (M⁺).

### Synthesis of 5-(3-methoxyphenyl)furan-2-carbonitrile

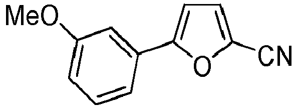

According to **TP1**, injection loop A and B (2.0 mL) were loaded with solutions of 2-furonitrile (3.0 mL; 0.69 M) and (Cy₂N)₂Zn·2LiCl (3.0 mL; 0.38 M in THF + 10 vol% DMPU; 0.55 equiv). After injection (flow rate of pump A and B: 1.00 mL/min) and metalation (residence time: 10 min; metalation temperature: 80 °C) the combined streams were collected in a flask containing 3-iodoanisole (257 mg; 1.10 mmol; 0.8 equiv), Pd(dba)₂ (16 mg; 2 mol%) and tfp (13 mg; 4 mol%) dissolved in THF (2 mL). The reaction mixture was further stirred overnight at 25 °C before it was quenched with water (30 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i-*hexane:EtOAc = 15:1) afforded the target compound as a yellow solid (153 mg; 0.77 mmol; 70%).
**m.p.** (°C): 86.5 - 88.3.
**¹H-NMR** (400 MHz, CDCl₃): δ / ppm = 7.36 - 7.25 (m, 2H), 7.23 - 7.22 (m, 1H), 7.14 (d, *J* = 3.7 Hz, 1H), 6.93 - 6.90 (m, 1H), 6.69 (d, *J* = 3.7 Hz, 1H), 3.85 (s, 3H).
**¹³C-NMR** (101 MHz, CDCl₃): δ / ppm = 160.1, 158.5, 130.1, 129.9, 125.1, 123.9, 117.4, 115.5, 111.9, 110.1, 106.3, 55.4.
**IR** (Diamond-ATR, neat): *ṽ* / cm⁻¹ = 3123, 3006, 2943, 2913, 2836, 2221, 1747, 1615, 1590, 1572, 1515, 1486, 1453, 1435, 1358, 1338, 1318, 1286, 1249, 1231, 1208, 1177, 1154, 1102, 1061, 1044, 1031, 994, 964, 896, 868, 836, 823, 796, 775, 688, 676, 622, 596, 568, 559.
**MS** (EI, 70 eV): m/z (%) = 200 (11), 199 (M⁺, 100), 156 (11).
**HRMS** (EI): calcd. for [C₁₂H₉NO₂]: 199.0633; found: 199.0628 (M⁺).

### Synthesis of 2,6-dichloro-9-(methoxymethyl)-8-(4-nitrophenyl)-9H-purine

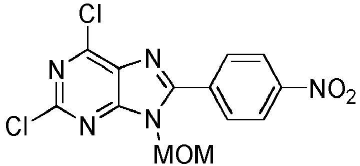

According to **TP1**, injection loop A and B (2.0 mL) were loaded with solutions of 2,6-dichloro-9-(methoxymethyl)-9H-purine (3.0 mL; 0.69 M) and (Cy₂N)₂Zn·2LiCl (3.0 mL; 0.38 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing 4-iodonitrobenzene (274 mg; 1.10 mmol; 0.8 equiv), Pd(dba)₂ (16 mg; 2 mol%) and tfp (13 mg; 4 mol%) dissolved in THF (2 mL). The reaction mixture was further stirred overnight at 25 °C before it was quenched with water (20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 5:1 + 0.5 vol% NEt₃) afforded the target compound as a yellow solid (236 mg; 0.67 mmol; 61 %).
**m.p.** (°C): 189.2 - 192.5.
**¹H-NMR** (400 MHz, CDCl₃): δ / ppm = 8.41 - 8.32 (m, 4H), 5.61 (s, 2H), 3.60 (s, 3H). **¹³C-NMR** (101 MHz, CDCl₃): δ / ppm = 155.4, 154.9, 153.6, 152.0, 149.6, 133.6, 131.0, 130.2, 124.2, 74.0, 58.1.
**IR** (Diamond-ATR, neat): *ṽ* / cm⁻¹ = 3092, 3038, 3011, 2938, 2850, 2823, 1601, 1581, 1564, 1518, 1506, 1482, 1447, 1403, 1367, 1341, 1318, 1300, 1260, 1223, 1202, 158, 1146, 1107, 1086, 1032, 1010, 967, 885, 868, 855, 808, 787, 752, 715, 708, 688, 678, 663.
**MS** (EI, 70 eV): m/z (%) = 353 (14), 325 (18), 324 (11), 323 (29), 322 (12), 45 (100).
**HRMS** (EI): calcd. for [C₁₃H₉Cl₂N₅O₃]: 353.0082; found: 353.0084 (M⁺).

### Synthesis of 2,6-dichloro-9-(methoxymethyl)-8-(4-methoxyphenyl)-9H-purine

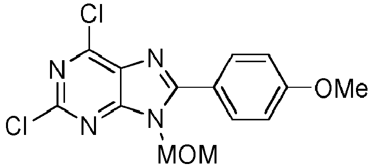

According to **TP1**, injection loop A and B (2.0 mL) were loaded with solutions of 2,6-dichloro-9-(methoxymethyl)-9H-purine (3.0 mL; 0.69 M) and (Cy₂N)₂Zn·2LiCl (3.0 mL; 0.38 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing 4-iodoanisole (257 mg; 1.10 mmol; 0.8 equiv), Pd(dba)₂ (16 mg; 2 mol%) and tfp (13 mg; 4 mol%) dissolved in THF (2 mL). The reaction mixture was further stirred overnight at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 5:1 + 0.5 vol% NEt₃) afforded the target compound as a yellow solid (366 mg; 1.08 mmol; 98%).
**m.p.** (°C): 163.8 - 165.6.
**¹H-NMR** (400 MHz, CDCl₃): δ / ppm = 8.04 (d, *J* = 9.0 Hz, 2H), 7.03 (d, *J* = 9.0 Hz, 2H), 5.55 (s, 2H), 3.87 (s, 3H), 3.56 (s, 3H).
**¹³C-NMR** (101 MHz, CDCl₃): δ / ppm = 162.5, 157.6, 155.8, 152.2, 150.1, 131.6, 130.4, 120.0, 114.6, 74.0, 57.8, 55.5.
**IR** (Diamond-ATR, neat): *ṽ* / cm⁻¹ = 3065, 3014, 2923, 2851, 1609, 1563, 1526, 1478, 1462, 1449, 1437, 1425, 1399, 1366, 1353, 1313, 1297, 1259, 1219, 1198, 1179, 1157, 1117, 1081, 1042, 1019, 963, 954, 882, 839, 801, 782, 773, 742, 707, 677, 665.
**MS** (EI, 70 eV): m/z (%) = 340 (45), 339 (M⁺, 13), 338 (68), 310 (24), 309 (22), 308 (37), 307 (27), 295 (12), 293 (12), 133 (15), 45 (100).
**HRMS** (EI): calcd. for [C₁₄H₁₂Cl₂N₄O₂]: 338.0337; found: 338.0332 (M⁺).

### Synthesis of 2,6-dichloro-8-(cyclohex-2-enyl)-9-(methoxymethyl)-9H-purine

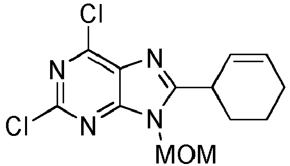

According to **TP1**, injection loop A and B (2.0 mL) were loaded with solutions of 2,6-dichloro-9-(methoxymethyl)-9H-purine (3.0 mL; 0.56 M) and (Cy₂N)₂Zn·2LiCl (3.0 mL; 0.31 M in THF + 10 vol% DMPU; 0.55 equiv). After injection (flow rate of pump A and B: 1.00 mL/min) and metalation (residence time: 10 min; metalation temperature: 25 °C) the combined streams were collected in a flask containing 3-bromocyclohexene (145 mg; 0.90 mmol; 0.8 equiv) and CuCN·2LiCl (0.11 mL; 10 mol%) dissolved in THF (2 mL) and cooled to 0 °C. The reaction mixture was stirred for further 1 h at 0 °C and overnight at 25 °C before it was quenched with sat. aq. NH₄Cl/NH₃ (10 vol%; 20 mL). The aq. layer was extracted with EtOAc (3×80 mL), the combined organic fractions were dried over anhydrous Na₂SO₄, filtrated and the solvent was removed *in vacuo.* Purification by flash column chromatography (silica gel; *i*-hexane:EtOAc = 5:1 + 0.5 vol% NEt₃) afforded the target compound as a yellow solid (220 mg; 0.70 mmol; 78%).
**m.p.** (°C): 97.1 - 99.1.
**¹H-NMR** (400 MHz, CDCl₃): δ / ppm = 6.02 - 5.97 (m, 1H), 5.75 - 5.72 (m, 1H), 5.58 (s, 2H), 3.93 - 3.86 (m, 1H), 3.37 (s, 3H), 2.23 - 2.10 (m, 3H), 2.03 - 1.92 (m, 2H), 1.74 - 1.63 (m, 1H).
**¹³C-NMR** (101 MHz, CDCl₃): δ/ppm = 162.5, 155.1, 152.4, 150.2, 130.8, 130.0, 124.3, 73.2, 57.4, 35.6, 28.1, 24.5, 21.2.
**IR** (Diamond-ATR, neat): *ṽ* / cm⁻¹ = 3030, 2935, 2836, 1734, 1591, 1560, 1503, 1465, 1448, 1435, 1390, 1355, 1314, 1294, 1266, 1239, 1224, 1196, 1181, 1139, 1126, 1084, 1044, 995, 970, 916, 902, 876, 847, 792, 749, 722, 682.
**MS** (EI, 70 eV): m/z (%) = 314 (14), 312 (20), 284 (14), 283 (16), 282 (19), 281 (21), 271 (13), 267 (22), 253 (14), 216 (12), 45 (100).
**HRMS** (EI): calcd. for [C₁₃H₁₄Cl₂N₄O]: 312.0545; found: 312.0540 (M⁺).

### Synthesis of 5-allyl-2,4,6-trichloropyrimidine

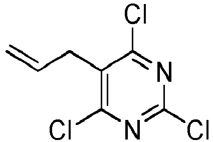

According to **TP1**, injection loop A and B (4.0 mL) were loaded with solutions of 2,4,6-trichloropyrimidine (5.0 mL; 0.49 M) and (Cy₂N)₂Zn·2LiCl (5.0 mL; 0.27 M in THF + 10 vol% DMPU; 0.55 equiv). After injection (flow rate of pump A and B: 1.00 mL/min) and metalation (residence time: 10 min; metalation temperature: 50 °C) the combined streams were collected in a flask containing allyl bromide (190 mg; 1.57 mmol; 0.8 equiv) and CuCN·2LiCl (0.20 mL; 10 mol%) dissolved in THF (2 mL) and cooled to 0 °C. The reaction mixture was stirred for further 1 h at 0 °C and 30 min at 25 °C before it was quenched with sat. aq. NH₄Cl/NH₃ (10 vol%; 20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 100:1 + 0.5 vol% NEt₃) afforded the target compound as a yellow oil (324 mg; 1.45 mmol; 92%).
**¹H-NMR** (400 MHz, CDCl₃): δ / ppm = 5.84 - 5.74 (m, 1H), 5.16 - 5.06 (m, 2H), 3.60 - 3.58 (m,2H).
**¹³C-NMR** (101 MHz, CDCl₃): δ / ppm = 163.0, 157.1, 130.2, 129.3, 118.5, 33.6.
**IR** (Diamond-ATR, neat): *ṽ* / cm⁻¹ = 3086, 2985, 1640, 1538, 1497, 1432, 1415, 1333, 1311, 1213, 1184, 1172, 1116, 1094, 989, 928, 908, 870, 834, 791, 741, 730.
**MS** (EI, 70 eV): m/z (%) = 310 (17), 286 (19), 284 (27), 228 (18), 226 (27), 224 (91), 222 (100), 201 (22), 189 (26), 188 (34), 187 (36), 186 (34), 153 (26), 152 (32), 151 (69), 150 (47), 126 (27), 125 (33), 90 (44), 83 (17), 57 (22), 55 (45), 44 (30), 43 (17), 43 (39), 41 (47).
**HRMS** (EI): calcd. for [C₇H₅Cl₃N₂]: 221.9518; found: 221.9517 (M⁺).

### Synthesis of ethyl 4-(3-bromo-5-fluoropyridin-4-yl)benzoate

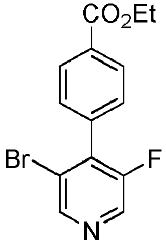

According to **TP1**, injection loop A and B (2.0 mL) were loaded with solutions of 3-bromo-5-fluoropyridine (3.0 mL; 0.56 M) and (Cy₂N)₂Zn·2LiCl (3.0 mL; 0.31 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 4-iodobenzoate (248 mg; 0.90 mmol; 0.8 equiv), Pd(dba)₂ (13 mg; 2 mol%) and tfp (10 mg; 4 mol%) dissolved in THF (2 mL). The reaction mixture was further stirred overnight at 25 °C before it was quenched with water (30 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 20:1 + 0.5 vol% NEt₃) afforded the target compound as a yellow oil (175 mg; 0.54 mmol; 60%).
**¹H-NMR** (400 MHz, CDCl₃): δ / ppm = 8.66 (s, 1H), 8.47 (s, 1H), 8.15 (d, *J* = 8.3 Hz, 2H), 7.42 (d, *J* = 8.1 Hz, 2H), 4.39 (q, *J* = 7.1 Hz, 2H), 1.39 (t, *J* = 7.1 Hz, 3H).
**¹³C-NMR** (101 MHz, CDCl₃): δ / ppm = 165.9, 156.3 (d, *J* = 261.5 Hz), 148.2 (d, *J* = 5.0 Hz), 137.2 (d, *J* = 15.1 Hz), 137.1 (d, *J* = 25.1 Hz), 135.9, 131.4, 129.7, 129.5 (d, *J* = 1.0 Hz), 121.2 (d, *J* = 2.0 Hz), 61.3, 14.3.
**IR** (Diamond-ATR, neat): ν̃ / cm⁻¹ = 3061, 2980, 2930, 2872, 1715, 1612, 1585, 1572, 1533, 1453, 1398, 1367, 1312, 1261, 1214, 1180, 1137, 1099, 1031, 1012, 964, 882, 854, 774, 754,740,702,668.
**MS** (EI, 70 eV): m/z (%) = 325 (27), 323 (26), 297 (49), 295 (53), 280 (95), 279 (16), 278 (100), 171 (51), 144 (37).
**HRMS** (EI): calcd. for [C₁₄H₁₁BrFNO₂]: 322.9957; found: 322.9952 (M⁺).

### Synthesis of 4-allyl-2,3,5-trichloropyridine

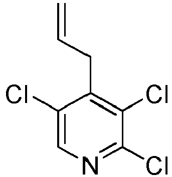

According to **TP1**, injection loop A and B (4.0 mL) were loaded with solutions of 2,3,5-trichloropyridine (5.0 mL; 0.49 M) and (Cy₂N)₂Zn·2LiCl (5.0 mL; 0.27 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing allyl bromide (190 mg; 1.57 mmol; 0.8 equiv) and CuCN·2LiCl (0.20 mL; 10 mol%) dissolved in THF (2 mL) and cooled to 0 °C. The reaction mixture was stirred for further 30 min at 0 °C and 1 h at 25 °C before it was quenched with sat. aq. NH₄Cl/NH₃ (10 vol%; 20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 100:1 + 0.5 vol% NEt₃) afforded the target compound as a colorless oil (338 mg; 1.52 mmol; 97%).
**¹H-NMR** (400 MHz, CDCl₃): δ / ppm = 8.24 (s, 1H), 5.86 - 5.76 (m, 1H), 5.14 - 5.06 (m, 2H), 3.71 - 3.68 (m, 2H).
**¹³C-NMR** (101 MHz, CDCl₃): δ/ppm = 148.2, 147.2, 146.0, 131.6, 131.3, 130.5, 118.3, 36.0.
**IR** (Diamond-ATR, neat): *ṽ* / cm⁻¹ = 3085, 2929, 1639, 1550, 1417, 1342, 1219, 1181, 1119, 1092, 1040, 989, 923, 888, 844, 808, 752, 718, 678.
**MS** (EI, 70 eV): m/z (%) = 225 (22), 223 (M⁺, 67), 221 (67), 188 (27), 187 (12), 186 (45), 185 (11), 153 (13), 152 (33), 151 (38), 150 (100), 123 (18), 114 (18), 63 (10).
**HRMS** (EI): calcd. for [C₈H₆Cl₃N]: 220.9566; found: 220.9568 (M⁺).

### Synthesis of ethyl 2-((2,3,5-trichloropyridin-4-yl)methyl)acrylate

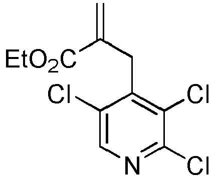

According to **TP1**, injection loop A and B (2.0 mL) were loaded with solutions of 2,3,5-trichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 2-(bromomethyl)acrylate (241 mg; 1.25 mmol; 0.8 equiv) and CuCN·2LiCl (0.16 mL; 10 mol%) dissolved in THF (2 mL) and cooled to 0 °C. The reaction mixture was stirred for further 30 min at 0 °C and 1 h at 25 °C before it was quenched with sat. aq. NH₄Cl/NH₃ (10 vol%; 20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i-*hexane:EtOAc = 100:1 + 0.5 vol% NEt₃) afforded the target compound as a colorless oil (236 mg; 0.80 mmol; 64%).
**¹H-NMR** (400 MHz, CDCl₃): δ / ppm = 8.29 (s, 1H), 6.21 (s, 1H), 4.99 (s, 1H), 4.25 (q, *J* = 7.1 Hz, 2H), 3.96 (s, 2H), 1.31 (t, *J* = 7.1 Hz, 3H).
**¹³C-NMR** (101 MHz, CDCl₃): δ / ppm = 165.9, 148.4, 146.6, 146.1, 134.5, 132.2, 132.0, 125.1, 61.4, 33.7, 14.2.
**IR** (Diamond-ATR, neat): *ṽ* / cm⁻¹ = 2982, 2936, 2907, 2360, 1712, 1634, 1552, 1527, 1465, 1421, 1368, 1343, 1278, 1252, 1216, 1191, 1173, 1133, 1094, 1024, 952, 905, 862, 813, 756, 694.
**MS** (EI, 70 eV): m/z (%) = 260 (49), 259 (10), 258 (73), 250 (14), 248 (13), 234 (11), 232 (66), 231 (12), 230 (100), 186 (13), 185 (12), 184 (18), 150 (12), 43 (46).
**HRMS** (EI): calcd. for [C₁₁H₁₀Cl₃NO₂]: 292.9777; found: 292.9769 (M⁺).

### Synthesis of 4-allyl-3,5-dichloropyridine

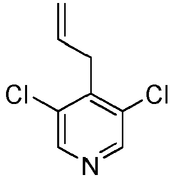

According to **TP1**, injection loop A and B (2.0 mL) were loaded with solutions of 3,5-dichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing allyl bromide (151 mg; 1.25 mmol; 0.8 equiv) and CuCN·2LiCl (0.16 mL; 10 mol%) dissolved in THF (2 mL) and cooled to 0 °C. The reaction mixture was stirred for further 30 min at 0 °C and 30 min at 25 °C before it was quenched with sat. aq. NH₄Cl/NH₃ (10 vol%; 20 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 200:1 + 0.5 vol% NEt₃) afforded the target compound as a colorless oil (205 mg; 1.09 mmol; 87%).
**¹H-NMR** (400 MHz, CDCl₃): δ / ppm = 8.42 (s, 2H), 5.89 - 5.79 (m, 1H), 5.12 - 5.03 (m, 2H), 3.67 - 3.64 (m, 2H).
**¹³C-NMR** (101 MHz, CDCl₃): δ / ppm = 147.6, 144.2, 132.8, 131.1, 117.8, 34.6.
**IR** (Diamond-ATR, neat): *ṽ* / cm⁻¹ = 3083, 3048, 3014, 2983, 2927, 2854, 1740, 1638, 1615, 1560, 1529, 1435, 1403, 1390, 1295, 1270, 1239, 1213, 1188, 1167, 1147, 1087, 1047, 1011, 989, 920, 886, 838, 826, 811, 792, 739, 689, 661.
**MS** (EI, 70 eV): m/z (%) = 191 (10), 189 (58), 188 (M⁺, 15), 187 (100), 186 (10), 155 (12), 153 (39), 152 (17), 151 (11), 117 (60), 116 (53), 115 (13), 89 (33), 63 (13), 43 (25).
**HRMS** (EI): calcd. for [C₈H₇Cl₂N]: 186.9956; found: 186.9950 (M⁺).

### Synthesis of ethyl 2-((3,5-dichloropyridin-4-yl)methyl)acrylate

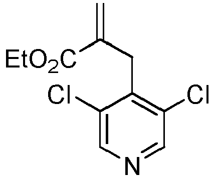

According to **TP1**, injection loop A and B (2.0 mL) were loaded with solutions of 3,5-dichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 2-(bromomethyl)acrylate (241 mg; 1.25 mmol; 0.8 equiv) and CuCN·2LiCl (0.16 mL; 10 mol%) dissolved in THF (2 mL) and cooled to 0 °C. The reaction mixture was stirred for further 30 min at 0 °C and 30 min at 25 °C before it was quenched with sat. aq. NH₄Cl/NH₃ (10 vol%; 20 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i-*hexane:EtOAc = 20:1 + 0.5 vol% NEt₃) afforded the target compound as a colorless solid (242 mg; 0.93 mmol; 74%).
**m.p.** (°C): 67.9 - 69.4.
**¹H-NMR** (400 MHz, CDCl₃): δ / ppm = 8.46 (s, 2H), 6.20 (s, 1H), 4.98 (s, 1H), 4.25 (q, *J* = 7.1 Hz, 2H), 3.92 (s, 2H), 1.31 (t, *J =* 7.1 Hz, 3H).
**¹³C-NMR** (101 MHz, CDCl₃): δ / ppm = 166.1, 147.6, 143.6, 134.9, 133.4, 125.0, 61.3, 32.3, 14.2.
**IR** (Diamond-ATR, neat): *ṽ* / cm⁻¹ = 3049, 2984, 2961, 2928, 2855, 2360, 1708, 1673, 1634, 1562, 1528, 1476, 1446, 1426, 1411, 1395, 1368, 1345, 1281, 1223, 1213, 1189, 1162, 1136, 1113, 1088, 1026, 953, 895, 870, 844, 819, 806, 792, 749, 675.
**MS** (EI, 70 eV): m/z (%) = 226 (33), 225 (12), 224 (94), 216 (12), 214 (17), 198 (32), 197 (12), 196 (100), 151 (15), 150 (21), 43 (23).
**HRMS** (EI): calcd. for [C₁₁H₁₁Cl₂NO₂]: 259.0167; found: 259.0165 (M⁺).

### Synthesis of ethyl 4-(2,5-dichloropyridin-4-yl)benzoate

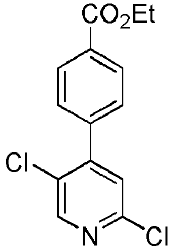

According to **TP1**, injection loop A and B (2.0 mL) were loaded with solutions of 2,5-dichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 4-iodobenzoate (345 mg; 1.25 mmol; 0.8 equiv), Pd(dba)₂ (18 mg; 2 mol%) and tfp (14 mg; 4 mol%) dissolved in THF (2 mL). The reaction mixture was further stirred for 2 h at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 20:1 + 0.5 vol% NEt₃) afforded the target compound as a colorless solid (327 mg; 1.10 mmol; 88%).
**m.p.** (°C): 111.8 - 113.2.
**¹H-NMR** (400 MHz, CDCl₃): δ / ppm = 8.45 (s, 1H), 8.14 (d, *J* = 8.5 Hz, 2H), 7.50 (d, *J* = 8.5 Hz, 2H), 7.31 (s, 1H), 4.40 (q, *J* = 7.1 Hz, 2H), 1.40 (t, *J* = 7.1 Hz, 3H).
**¹³C-NMR** (101 MHz, CDCl₃): δ / ppm = 165.9, 149.9, 149.7, 149.5, 139.6, 131.4, 129.8, 129.1, 128.9, 125.4, 61.3, 14.3.
**IR** (Diamond-ATR, neat): *ṽ* / cm⁻¹ = 3075, 3045, 2993, 2983, 2938, 2911, 2870, 1711, 1665, 1646, 1611, 1573, 1519, 1508, 1480, 1468, 1448, 1422, 1406, 1365, 1337, 1314, 1278, 1213, 1182, 1146, 1127, 1119, 1102, 1081, 1026, 940, 915, 907, 875, 860, 828, 773, 750, 738,726,703,655.
**MS** (EI, 70 eV): m/z (%) = 295 (10), 269 (12), 267 (20), 252 (23), 250 (32), 70 (14), 61 (18), 45 (16), 43 (100).
**HRMS** (EI): calcd. for [C₁₄H₁₁Cl₂NO₂]: 295.0167; found: 295.0158 (M⁺).

### Synthesis of 2,5-dichloro-4-(4-nitrophenyl)pyridine

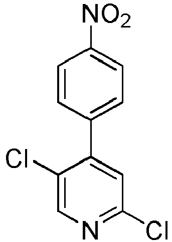

According to **TP1**, injection loop A and B (2.0 mL) were loaded with solutions of 2,5-dichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing 4-iodonitrobenzene (311 mg; 1.25 mmol; 0.8 equiv), Pd(dba)₂ (18 mg; 2 mol%) and tfp (14 mg; 4 mol%) dissolved in THF (2 mL). The reaction mixture was further stirred for 2 h at 25 °C before it was quenched with water (30 mL). The aq. layer was extracted with EtOAc (3×70 mL), the combined organic fractions were dried over anhydrous Na₂SO₄, filtrated and the solvent was removed *in vacuo.* Purification by flash column chromatography (silica gel; *i*-hexane:EtOAc = 10:1 + 0.5 vol% NEt₃) afforded the target compound as a yellow solid (264 mg; 0.98 mmol; 78%).
m.p. (°C): 158.3 - 160.1.
**¹H-NMR** (400 MHz, CDCl₃): δ / ppm = 8.49 (s, 1H), 8.34 (d, *J* = 8.9 Hz, 2H), 7.63 (d, *J* = 8.9 Hz, 2H), 7.33 (s, 1H).
**¹³C-NMR** (101 MHz, CDCl₃): δ / ppm = 150.1, 149.9, 148.4, 148.1, 141.5, 130.0, 128.9, 125.3, 123.9.
**IR** (Diamond-ATR, neat): *ṽ* / cm⁻¹ = 3106, 3056, 2930, 2851, 1740, 1651, 1621, 1602, 1573, 1513, 1495, 1446, 1422, 1397, 1351, 1336, 1319, 1294, 1281, 1251, 1239, 1213, 1190, 1143, 1116, 1047, 1024, 1009, 985, 925, 919, 894, 857, 826, 759, 735, 727, 715, 696, 659.
**MS** (EI, 70 eV): m/z (%) = 270 (80), 268 (100), 241 (18), 140 (33), 240 (24), 238 (35), 224 (12), 222 (23), 212 (26), 196 (17), 195 (14), 189 (26), 187 (56), 168 (37), 167 (30), 166 (48), 162 (12), 160 (29), 140 (13), 74 (14), 61 (15), 45 (17), 44 (63), 43 (87).
**HRMS** (EI): calcd. for [C₁₁H₆C₁₂N₂O₂]: 267.9806; found: 267.9808 (M⁺).

### Synthesis of ethyl 4-(2,3-dichloropyridin-4-yl)benzoate

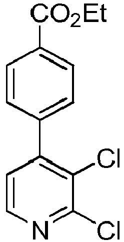

According to **TP1**, injection loop A and B (2.0 mL) were loaded with solutions of 2,3-dichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing ethyl 4-iodobenzoate (345 mg; 1.25 mmol; 0.8 equiv), Pd(dba)₂ (18 mg; 2 mol%) and tfp (14 mg; 4 mol%) dissolved in THF (2 mL). The reaction mixture was further stirred for 2 h at 25 °C before it was quenched with water (30 mL). The aq. layer was extracted with EtOAc (3×60 mL), the combined organic fractions were dried over anhydrous Na₂SO₄, filtrated and the solvent was removed *in vacuo.* Purification by flash column chromatography (silica gel; *i*-hexane:EtOAc = 10:1 + 0.5 vol% NEt₃) afforded the target compound as a colorless solid (242 mg; 0.82 mmol; 66%).
**m.p.** (°C): 117.9 - 119.8.
**¹H-NMR** (400 MHz, CDCl₃): δ / ppm = 8.32 (d, *J* = 4.9 Hz, 1H), 8.13 (d, *J* = 8.5 Hz, 2H), 7.48 (d, *J =* 8.5 Hz, 2H), 7.19 (d, *J =* 4.9 Hz, 1H), 4.39 (q, *J* = 7.1 Hz, 2H), 1.39 (t, *J* = 7.1 Hz, 3H). **¹³C-NMR** (101 MHz, CDCl₃): δ / ppm = 165.9, 150.7, 150.1, 146.7, 140.9, 131.2, 129.7, 128.8, 128.8, 124.3, 61.3, 14.3.
**IR** (Diamond-ATR, neat): *ṽ* / cm⁻¹ = 3062, 2988, 2964, 2925, 2854, 1938, 1711, 1671, 1651, 1611, 1574, 1515, 1470, 1453, 1443, 1397, 1360, 1312, 1287, 1278, 1246, 1196, 1184, 1154, 1137, 1128, 1101, 1063, 1035, 1018, 964, 876, 867, 850, 801, 770, 748, 703, 671.
**MS** (EI, 70 eV): m/z (%) = 297 (18), 295 (28), 267 (60), 254 (14), 253 (11), 252 (66), 251 (16), 250 (100), 224 (10), 222 (16), 189 (10), 187 (28), 152 (10), 151 (10), 61 (11), 45 (12), 43 (59).
**HRMS** (EI): calcd. for [C₁₄H₁₁Cl₂NO₂]: 295.0167; found: 295.0157 (M⁺).

### Synthesis of 2,3-dichloro-4-(3-nitrophenyl)pyridine

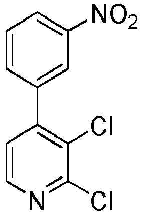

According to **TP1**, injection loop A and B (2.0 mL) were loaded with solutions of 2,3-dichloropyridine (3.0 mL; 0.78 M) and (Cy₂N)₂Zn·2LiCl (3.0 mL; 0.43 M in THF + 10 vol% DMPU; 0.55 equiv). After injection (flow rate of pump A and B: 1.00 mL/min) and metalation (residence time: 10 min; metalation temperature: 60 °C) the combined streams were collected in a flask containing 3-iodonitrobenzene (311 mg; 1.25 mmol; 0.8 equiv), Pd(dba)₂ (18 mg; 2 mol%) and tfp (14 mg; 4 mol%) dissolved in THF (2 mL). The reaction mixture was further stirred for 2 h at 25 °C before it was quenched with water (30 mL). The aq. layer was extracted with EtOAc (3×80 mL), the combined organic fractions were dried over anhydrous Na₂SO₄, filtrated and the solvent was removed *in vacuo.* Purification by flash column chromatography (silica gel; *i*-hexane:EtOAc = 5:1 + 0.5 vol% NEt₃) afforded the target compound as a yellow solid (209 mg; 0.78 mmol; 62%).
**m.p.** (°C): 165.8 - 167.5.
**¹H-NMR** (400 MHz, CDCl₃): δ / ppm = 8.38 (d, *J* = 4.9 Hz, 1H), 8.34 - 8.30 (m, 2H), 7.79 - 7.76 (m, 1H), 7.70 - 7.66 (m, 1H), 7.23 (d, *J* = 4.9 Hz, 1H).
**¹³C-NMR** (101 MHz, CDCl₃): δ / ppm = 151.0, 148.4, 148.3, 147.1, 138.1, 134.8, 129.7, 128.9, 124.2, 124.1, 123.9.
**IR** (Diamond-ATR, neat): *ṽ* / cm⁻¹ = 3066, 2924, 2860, 2360, 2340, 1614, 1572, 1520, 1483, 1443, 1427, 1367, 1344, 1310, 1302, 1276, 1251, 1222, 1196, 1175, 1143, 1119, 1105, 1093, 1065, 1039, 1024, 999, 972, 951, 917, 894, 850, 843, 813, 808, 766, 751, 736, 689, 682.
**MS** (EI, 70 eV): m/z (%) = 272 (11), 270 (66), 268 (100), 224 (24), 222 (35), 189 (17), 187 (53), 186 (11), 160 (11), 152 (14), 151 (16), 126 (12), 125 (14), 42 (32).
**HRMS** (EI): calcd. for [C₁₁H₆C₁₂N₂O₂]: 267.9806; found: 267.9811 (M⁺).

### Zincation of 3,6-dichloropyridazine using (Cy₂N)₂Zn·2LiCl in THF/DMPU or THF/TMU or THF followed by the reaction with iodine

**(Cy₂N)₂Zn·2LiCl** (solution in THF/DMPU = 10:1) was prepared by slow addition of *n*-BuLi (8.2 mL; 20 mmol; 2.5 M in hexane) to a solution of Cy₂NH (3.7 g; 4.1 mL; 20 mmol) in THF (20 mL) at -40 °C and slowly warmed to -20 °C within 30 min. ZnCl₂ (10 mL; 10 mmol; 1.00 M in THF) was added at -20 °C and the solution was slowly warmed to 25 °C within 2 h. All solvents were removed *in vacuo* and the remaining residue was dissolved under vigorous stirring in freshly distilled THF and 10 vol% DMPU. The solvent mixture was added in small portions, and the addition was stopped when a clear solution was obtained to obtain a maximum concentration of the Zn complex. The concentration was determined by titration against benzoic acid in THF using 4-(phenylazo)diphenylamine as indicator.

**(Cy₂N)₂Zn-2LiCl** (solution in THF/TMU = 10:1) was prepared by slow addition of n-BuLi (8.2 mL; 20 mmol; 2.5 M in hexane) to a solution of Cy₂NH (3.7 g; 4.1 mL; 20 mmol) in THF (20 mL) at -40 °C and slowly warmed to -20 °C within 30 min. ZnCl₂ (10 mL; 10 mmol; 1.00 M in THF) was added at -20 °C and the solution was slowly warmed to 25 °C within 2 h. All solvents were removed *in vacuo* and the remaining residue was dissolved under vigorous stirring in freshly distilled THF and 10 vol% TMU. The solvent mixture was added in small portions, and the addition was stopped when a clear solution was obtained to obtain a maximum concentration of the Zn complex. The concentration was determined by titration against benzoic acid in THF using 4-(phenylazo)diphenylamine as indicator.

**(Cy₂N)₂Zn-2LiCl** (solution in THF; Reference Sample) was prepared by slow addition of *n-*BuLi (8.2 mL; 20 mmol; 2.5 M in hexane) to a solution of Cy₂NH (3.7 g; 4.1 mL; 20 mmol) in THF (20 mL) at -40 °C and slowly warmed to -20 °C within 30 min. ZnCl₂ (10 mL; 10 mmol; 1.00 M in THF) was added at -20 °C and the solution was slowly warmed to 25 °C within 2 h. All solvents were removed *in vacuo* and the remaining residue was dissolved under vigorous stirring in freshly distilled THF. The solvent was added in small portions, and the addition was stopped when a clear solution was obtained to obtain a maximum concentration of the Zn complex. The concentration was determined by titration against benzoic acid in THF using 4-(phenylazo)diphenylamine as indicator.

According to **TP1**, the flow system (FlowSyn, Uniqsis) was dried by flushing it with dry THF (flow rate of all pumps: 1.00 mL/min; run-time: 30 min). Injection loop A (4.0 mL) was loaded with 3,6-dichloropyridazine (0.15 - 0.65 M in dry THF; 5.0 mL) and injection loop B (4.0 mL) was loaded with (Cy₂N)₂Zn·2LiCl (0.08 - 0.36 M; in dry THF/DMPU; dry THF/TMU; dry THF; 0.55 equiv; 5.0 mL). The solutions were simultaneously injected into separate THF streams (pump A and B, flow rates: 1.00 mL/min) respectively, before they were mixed in a coiled reactor followed by a tube (20.4 mL in total; residence time: 10 min; 60 °C). The combined streams were collected in a flame-dried, argon flushed 25 mL flask equipped with a magnetic stirrer and a septum containing iodine (2.0 equiv) dissolved in dry THF (2 mL). Then the reaction mixture was further stirred for 60 min at 25 °C. The completion of the reaction was checked by GC analysis of reaction aliquots quenched with concentrated aqueous NH₄Cl solution and using undecane as an internal standard. The results are summarized in the following table.

| Base | (Cy₂N)₂Zn·2LiCl (in THF/DMPU 10:1; 0.36 M) | (Cy₂N)₂Zn·2LiCl (in THF/TMU 10:1; 0.34 M) | (Cy₂N)₂Zn·2LiCl (in THF; 0.08 M; Reference Sample) |
|---|---|---|---|
| conversion | **91%** | **63%** | **38%** |

### Zincation of 3,6-dichloropyridazine in a continuous flow reactor using TMP₂Zn·2LiCi in THF/DMPU or THF followed by the reaction with iodine

**TMP₂Zn·2LiCl** (solution in THF/DMPU = 10:1) was prepared by slow addition of n-BuLi (8.2 mL; 20 mmol; 2.5 M in hexane) to a solution of TMPH (2.9 g; 3.4 mL; 20 mmol) in THF (20 mL) at -40 °C and slowly warmed to -20 °C within 30 min. ZnCl₂ (10 mL; 10 mmol; 1.00 M in THF) was added at -20 °C and the solution was slowly warmed to 25 °C within 2 h. All solvents were removed *in vacuo* and the remaining residue was dissolved under vigorous stirring in freshly distilled THF and 10 vol% DMPU. The solvent mixture was added in small portions, and the addition was stopped when a clear solution was obtained to obtain a maximum concentration of the Zn complex. The concentration was determined by titration against benzoic acid in THF using 4-(phenylazo)diphenylamine as indicator.

**TMP₂Zn·2LiCi** (solution in THF; Reference Sample) was prepared by slow addition of *n*-BuLi (8.2 mL; 20 mmol; 2.5 M in hexane) to a solution of TMPH (2.9 g; 3.4 mL; 20 mmol) in THF (20 mL) at -40 °C and slowly warmed to -20 °C within 30 min. ZnCl₂ (10 mL; 10 mmol; 1.00 M in THF) was added at -20 °C and the solution was slowly warmed to 25 °C within 2 h. All solvents were removed *in vacuo* and the remaining residue was dissolved under vigorous stirring in freshly distilled THF. The solvent was added in small portions, and the addition was stopped when a clear solution was obtained to obtain a maximum concentration of the Zn complex. The concentration was determined by titration against benzoic acid in THF using 4-(phenylazo)diphenylamine as indicator.

In accordance with **TP1**, the flow system (FlowSyn, Uniqsis) was dried by flushing it with dry THF (flow rate of all pumps: 1.00 mL/min; run-time: 30 min). Injection loop A (4.0 mL) was loaded with 3,6-dichloropyridazine (0.58 - 0.69 M in dry THF; 5.0 mL) and injection loop B (4.0 mL) was loaded with TMP₂Zn·2LiCl (0.32 - 0.38 M; in dry THF/DMPU; dry THF; 0.55 equiv; 5.0 mL). The solutions were simultaneously injected into separate THF streams (pump A and B, flow rates: 1.00 mL/min) respectively, before they were mixed in a coiled reactor followed by a tube (20.4 mL in total; residence time: 10 min; 60 °C). The combined streams were collected in a flame-dried, argon flushed 25 mL flask equipped with a magnetic stirrer and a septum containing iodine (2.0 equiv) dissolved in dry THF (2 mL). Then the reaction mixture was further stirred for 60 min at 25 °C. The completion of the reaction was checked by GC analysis of reaction aliquots quenched with concentrated aqueous NH₄Cl solution and using undecane as an internal standard. The results are summarized in the following table:

| Base | TMP₂Zn·2LiCl (in THF/DMPU 10:1; 0.38 M) | TMP₂Zn·2LiCl (in THF; 0.32 M, Reference Sample) |
|---|---|---|
| conversion | **85%** | **65%** |

Fig. 1 is a schematic representation of a flow system as used in typical procedure 1 (TP 1) in the examples section.

Fig. 2 is a schematic representation of a flow system as used in typical procedure 2 (TP 2) in the examples section.

## Claims

1. An organozinc composition comprising:
(a) a zinc complex, wherein a zinc atom carries two identical or different amide ligands of formula (I), which amide ligands are bound to the zinc atom via their nitrogen atom :
R^{a}R^{b}N (I)
wherein
R^{a} and R^{b} are independently selected from a saturated C₁₋₁₀ hydrocarbon group,
and wherein optionally
one or more of the carbon atoms in the hydrocarbon group may be replaced by a silicon atom each;
a group R^{a} and a group R^{b} may be connected in the same ligand R^{a}R^{b}N to form a 5 to 8 membered ring together with the nitrogen atom to which they are attached; and/or
a group R^{a} or R^{b} of one amide ligand may be connected with a group R^{a} or R^{b} of the other amide ligand, such that a bidentate ligand comprising two amide groups is formed;
(b) a lithium salt of the formula LiX,
wherein
X is an anion selected from the group consisting of H-, F⁻, Cl⁻, Br⁻, I⁻, SCN⁻, CN⁻, NCO⁻, HalOₘ⁻, wherein Hal is selected from Cl, Br, and I and m is 3 or 4, NOp⁻, wherein p is 2 or 3, BF₄⁻, PF₆⁻, R'O⁻, R'C(O)O⁻, R'S⁻, R'C(O)S⁻, R'C(S)O⁻, R'C(S)S⁻, R'R"N⁻, R'(R"O)P(O)O⁻, R'O(R"O)P(O)O⁻, R'R"P⁻, R'SO₃⁻, R'SO₂S⁻, and R'OSO₃⁻, wherein R' is H or an organic group selected from aryl, heteroaryl, alkaryl, alkyl, cycloalkyl, alkenyl and alkynyl, which organic group may be optionally substituted, wherein R" is H or an organic group selected from aryl, heteroaryl, alkaryl, alkyl, cycloalkyl, alkenyl and alkynyl, which organic group may be optionally substituted, and wherein two suitable organic groups R' and R" may be connected to form a ring together with the atoms to which they are attached;
(c) a solvent S1 or a solvent mixture comprising at least one solvent S1, wherein S1 is a solvent of formula (II) : 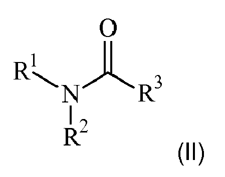 wherein
R¹ is selected from H and a saturated C₁₋₁₀ hydrocarbon group;
R² is a saturated C₁₋₁₀ hydrocarbon group;
and R³ is selected from NR⁴R⁵, H and R⁶, wherein R⁴ is selected from H and a saturated C₁₋₁₀ hydrocarbon group, R⁵ is a saturated C₁₋₁₀ hydrocarbon group and R⁶ is a saturated C₁₋₁₀ hydrocarbon group;
and wherein optionally
R² and R⁵ or R² and R⁶ may be connected to form a 5 to 8 membered ring together with the atoms to which they are attached.

2. The organozinc composition according to claim 1, wherein the zinc complex and the lithium salt form a compound of the following formula (Ia):
(R^{a}R^{b}N)₂Zn · 2LiX (Ia),
wherein R^{a}, R^{b} and X are defined as in claim 1.

3. The organozinc composition according to claim 1 or 2, wherein R^{a} and R^{b} are independently selected from cyclohexyl, isopropyl, trimethylsilyl, and *t*-butyl.

4. The organozinc composition according to any of claims 1 to 3, wherein X is selected from Cl⁻, Br⁻, I⁻, SCN⁻, CN⁻, NCO⁻, tosylate, mesylate, and R'O⁻, wherein R' is C₁₋₄ alkyl.

5. The organozinc composition according any of claims 1 to 4, wherein S1 is selected from the group consisting of 1,3-dimethyl-3,4,5,6-tetrahydro-2(1*H*)-pyrimidinone (DMPU), *N,N,N*'*,N*'-tetramethylurea, *N*-methyl-2-pyrrolidone (NMP), *N*-ethyl-2-pyrrolidone (NEP), and dimethylformamide (DMF).

6. The organozinc composition according to any of claims 1 to 5, which comprises a solvent mixture comprising at least one solvent S1 and a further solvent S2 different from S1, wherein S1 is contained in the solvent mixture in an amount of 3 vol% or more and 50 vol% or less, based on the total volume of solvents contained in the composition.

7. The organozinc composition according to claim 6, wherein the solvent S2 is selected from the group consisting of acetonitrile and ether solvents.

8. The organozinc composition of any of claims 1 to 7, wherein the concentration of the zinc complex in the organozinc composition is from 0.10 to 1.10 mol/l in terms of the molar amount of zinc contained in the organozinc composition.

9. A process for the preparation of the organozinc composition as defined in any one of claims 1 to 8, said process comprising the steps of :
(a) reacting an amine of the formula NHR^{a}R^{b}, wherein R^{a} and R^{b} are defined as in the preceding claims,
and a lithium base or metallic lithium to obtain an amide R^{a}R^{b}NLi; and
(b) reacting the amide with a Zn salt of the formula ZnX₂, wherein X is defined as in the preceding claims
to obtain a composition comprising a zinc complex wherein a zinc atom carries two identical or different amide ligands of formula (I) as defined in the preceding claims, and a lithium salt of the formula LiX;
wherein
either at least the reaction of step b) is carried out in a solvent S1 or a solvent mixture comprising at least one solvent S1 as defined in the preceding claims;
or wherein a solvent present in the reaction mixture obtained from reaction step b) is replaced by a solvent S1 or a solvent mixture comprising at least one solvent S1 as defined in the preceding claims.

10. A process for the preparation of the organozinc composition as defined in any one of claims 1 to 8, said process comprising the steps of :
(a) providing an amine of the formula NXR^{a}R^{b}, wherein X, R^{a} and R^{b} are defined as in the preceding claims,
(b) reacting the amine of the formula NXR^{a}R^{b} with metallic zinc, preferably in the form of zinc powder,
(c) adding LiX prior to, during or after the reaction of step (b), wherein X is defined as in the preceding claims,
to obtain a composition comprising a zinc complex wherein a zinc atom carries two identical or different amide ligands of formula (I) as defined in the preceding claims, and a lithium salt of the formula LiX;
wherein
either at least the reaction of step (b) is carried out in a solvent S1 or a solvent mixture comprising at least one solvent S1 as defined in the preceding claims;
or wherein a solvent present in the reaction mixture obtained from reaction step (b) is replaced by a solvent S1 or a solvent mixture comprising at least one solvent S1 as defined in the preceding claims.

11. A process for the preparation of an organozinc compound of formula (IIIa) and/or (IIIb),
R^{c}-Zn-R^{c} (IIIa)
R^{c}-Zn-NR^{a}R^{b} (IIIb)
wherein
R^{c} is an organic group bound to the Zn atom via a carbon atom, and wherein the two groups R^{c} in formula (IIIa) are identical or different,
and R^{a} and R^{b} are defined as in the preceding claims,
said process comprising the step of reacting
(i) an organozinc composition of any of claims 1 to 8 with
(ii) an organic compound of the formula R^{c}-H, wherein R^{c} is an organic group and wherein the hydrogen atom -H is bound to a carbon atom.

12. The process in accordance with claim 11, wherein R^{c} in formula (IIIa) or (IIIb) is selected from
(i) an organic group comprising an aryl or heteroaryl moiety which is bound to the Zn atom via a carbon atom contained as a ring member in the aryl or heteroaryl moiety, and wherein the hydrogen atom -H in the compound of formula R⁷-H is bound to a carbon atom contained as a ring member in the aryl or heteroaryl moiety;
(ii) an organic group comprising a benzyl or heterobenzyl moiety which is bound to the Zn atom via a methylene group attached to an aryl or heteroaryl group, and wherein the hydrogen atom -H in the compound of formula R^{c}-H is bound to a methylene group attached to an aryl or heteroaryl group;
(iii) an organic group comprising a carbonyl group (-C(O)-), in particular a ketone group or an ester group, which is bound to the Zn atom via a carbon atom in α-position relative to the carbonyl group, and wherein the hydrogen atom -H in the compound of formula R^{c}-H is bound to a carbon atom in α-position relative to a carbonyl group; and
(iv) an organic group comprising a C-C double bond or triple bond which organic group is bound to the Zn atom via a carbon atom involved in the C-C double bond or triple bond, and wherein the hydrogen atom -H in the compound of formula R^{c}-H is bound to a carbon atom involved in the C-C double bond or triple bond.

13. A coupling process comprising the steps of:
(i) preparing an organozinc compound of formula (IIIa) and/or (IIIb), in accordance with the process of claim 11 or 12; and
(ii) reacting the organozinc compound of formula (IIIa) and/or (IIIb), with an electrophil E or a reactant comprising an electrophilic moiety E.

14. A process for the formation of an organometallic compound comprising a group R^{c} bound to a metal atom M, wherein R^{c} is defined as in claim 11 or 12, and M is a metal or semi-metal other than Zn, said process comprising the steps of:
(i) preparing an organozinc compound of formula (IIIa) and/or (IIIb), preferably (IIIa), in accordance with the process of claim 11 or 12; and
(ii) reacting the organozinc compound of formula (IIIa) and/or (IIIb), preferably (IIIa), in a transmetalation reaction with M or a metal salt or an organometallic compound comprising M.

15. Use of the organozinc composition as defined in any of claims 1 to 8 as a metalation reagent for the metalation of an organic compound.
